(19) Europäisches Patentamt
European Patent Office
Office européen des brevets

(11) Veröffentlichungsnummer: **0 326 134**
**A1**

(12) # EUROPÄISCHE PATENTANMELDUNG

(21) Anmeldenummer: **89101353.4**

(22) Anmeldetag: **26.01.89**

(51) Int. Cl.⁴: **C07D 221/04 , C07D 221/16 , A61K 31/435**

(30) Priorität: **26.01.88 SU 4414565**

(43) Veröffentlichungstag der Anmeldung:
**02.08.89 Patentblatt 89/31**

(84) Benannte Vertragsstaaten:
**AT BE CH DE ES FR GB GR IT LI LU NL SE**

(71) Anmelder: **NAUCHNO-ISSLEDOVATELSKY INSTITUT TEKHNOLOGII I BEZOPASNOSTI LEKARSTVENNYKH SREDSTV**
**Ulitsa Kirova, 23**
**Poselok Staraya Kupavna Noginsky Raion**
**Moskovskaya Oblast(SU)**

Anmelder: **GOSUDARSTVENNY NAUCHNO-ISSLEDOVATELSKY I PROEKTNY INSTITUT AZOTNOI PROMYSHLENNOSTI I PRODUKTOV ORGANICHESKOGO SINTEZA**
**Ulitsa Chkalova, 50**
**Moskau(SU)**

(72) Erfinder: **Lavretskaya, Elionora Fishelevna**
**-(SU)**
Erfinder: **Upadysheva, Alexandra Vladimirovna**
**ulitsa Rozy Ljuxemburg, 4, kv. 59**
**Moskovskaya oblast, Khimki(SU)**
Erfinder: **Sukhanova, Svetlana Alexeevna**
**ulitsa Generala Dementieva, 7, kv. 63**
**Moskovskaya oblast, Monino(SU)**
Erfinder: **Grigorieva, Natalya Danilovna**
**prospekt Mira, 24, kv. 10**
**Moscow(SU)**
Erfinder: **Robakidze, Tatyana Nikolaevna**
**poselok Staraya Kupavna uli. Bolshaya Moskovskaya**
**138,kv.13 Moskovsk. obl. Noginsky raion(SU)**
Erfinder: **Demidova, Tamara Vasilievna**
**ulitsa Pushkinskaya, 9, kv. 70**
**Moscow(SU)**
Erfinder: **Mufazalova, Tatyana Alexeevna**
**poselok Staraya Kupavna mikroraion, 13, kv. 44**
**Moskovskaya oblast Noginsky raion(SU)**
Erfinder: **Batalova, Irina Pavlovna**
**poselok Staraya Kupavna ulitsa Matrosova 26, kv. 42 Moskovsk. obl. Noginsky raion(SU)**
Erfinder: **Naryshkova, Nelli Alexeevna**
**Troitsk mikroraion, 34, kv. 72**
**Moskovskaya oblast Podolsky raion(SU)**
Erfinder: **Kemenova, Vera Alexeevna**
**Shmitovsky proezd, 12, kv. 62**
**Moscow(SU)**
Erfinder: **Antonian, Semen Gurgenovich**
**ulitsa Fomichevoi 8, korpus 2, kv. 224**
**Moscow(SU)**
Erfinder: **Volkova, Ljubov Ivanovna**
**ulitsa Karbysheva 7, korpus 5, kv. 88**
**Moskovskaya oblast Balashikha(SU)**
Erfinder: **Libenzon, Rakhil Evelievna**
**Nagatinskaya naberezhnaya, 48/2, kv. 211**
**Moscow(SU)**
Erfinder: **Znamenskaya, Anna Pavlovna**
**Rizhsky proezd, 7, kv. 21**
**Moscow(SU)**
Erfinder: **Sarkisian, David Ashotovich**
**poselok Staraya Kupavna mikroraion 11, kv. 77**
**Moskovskaya oblast Noginsky raion(SU)**
Erfinder: **Berezovskaya, Irina Vladimirovna**
**poselok Staraya Kupavna mikroraion, 11, kv. 7**
**Moskovskaya oblast Noginsky raion(SU)**
Erfinder: **Dudenas, Genrikas Eduardovich**
**ulitsa Solomeyas Neris, 32, kv. 4**
**Kaunas(SU)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**



Xerox Copy Centre

(54) **4-Amino-2,3-disubst.-6,7-dihydro-5H-1-pyrindin-Derivate, Verfahren zu ihrer Herstellung und Arzneimittelpräparate auf ihrer Basis.**

(57) Beschrieben werden neue 4-Amino-2,3-disubst.-6,7-dihydro-5H-1-pyrindinderivate, und zwar die bicyclischen Basen sowie die Salze der tri- und bicyclischen Basen, Verfahren zu ihrer Herstellung und Arzneimittelpräparate, die als Wirkstoff solche Verbindungen enthält.

Die erfindungsgemäßen Verbindungen besitzen insbesonders die Fähigkeit, die Erregung im Nerven- und Muskelsystem zu stimulieren, das Gedächtnis wiederherzustellen und eine antiarrhythmische und analgetische Wirkung auszuüben.

# 4-AMINO-2,3-DISUBST.-6,7-DIHYDRO-5H-1-PYRINDIN-DERIVATE, VERFAHREN ZU IHRER HERSTELLUNG UND ARZNEIMITTELPRÄPARATE AUF IHRER BASIS

Die Erfindung betrifft die organische Chemie, und zwar die neuen Verbindungen der 4-Amino-2,3-disubst.-6,7-dihydro-5H-1-pyrindinderivate, die als Arzneimittel verwendet werden und insbesondere die Fähigkeit aufweisen, gleichzeitig Erregungen im Nerven- und Muskelsystem zu stimulieren, das Gedächtnis wiederherzustellen, sowie antiarrhythmische und analgetische Wirkung auszuüben, und die in der Medizin als Wirkstoff von Arzneimitteln verwendet werden können.

Anwendungsgebiet der erfindungsgemäßen Gruppe von Verbindungen sind zum Beispiel:

1. eine Gruppe von Erkrankungen, die mit Gedächtnisstörungen einhergehen, und zwar die Alzheimer-Krankheit und andere Arten seniler Demenz; minimale Hirndysfunktion bei Kindern; organische Hirnaffektionen unterschiedlicher Genese;

2. Affektionen des peripheren Nervensystems: Neuropathien, Myelopolyradikuloneuriten, Neuriten, darunter Neuriten der Gehörnerven;

3. Myasthenie, MS und andere demyelinisierende Erkrankungen des Nervensystems;

4. für die geburtsfördernde Wirkung in der Geburtshilfepraxis;

5. völlig überraschend hat sich herausgestellt, daß die angeführten Verbindungen auch noch eine antiarrhythmische Wirkung bei Arrhythmien zeigen, die sich nach dem Typ "reentry" entwickeln.

Bekannt ist das Arzneimittel Takrin® (THA), das als Wirkstoff 2,3,4,5-Tetrahydro-9-amino-acridin enthält (s. Summers et al. "ORAL tetrahydroaminoacridine in longtermtreatment of senile dementia Alzheimer type" Journal of Medicine, 315, 1986, S. 1241-1245). Als Cholinesteraseinhibitor wurde es in der medizinischen Praxis in verschiedenen Ländern verwendet. Dabei wurden Anticurare-, antiarrhythmische und Antimorphineigenschaften festgestellt. Aufgrund seiner schwachen klinischen Effektivität hat es jedoch keine breite Anwendung gefunden. Bis 1986 wurde THA praktisch nicht angewandt.

Den erfindungsgemäßen Verbindungen am nächsten steht ein Arzneimittel, welches als Wirkstoff 9-Amino-2,3,5,6,7,8-hexahydro-1H-cyclopenta(b)chinolinhydrochloridhydrat (Amyridin®) enthält (s. US-PS 4550113).

Die Wirkungsweise dieser Präparate ist am eingehendsten in den Veröffentlichungen der Zeitschrift "Chimiko-farmacevtičeskij žurnal", Nr. 2, 1982, S. 249 - 251, und Nr. 2, 1977, S. 40 - 44 beschrieben.

Gegenwärtig hat man es im Zusammenhang mit dem Anstieg des Anteils der älteren und senilen Menschen an der Bevölkerung sämtlicher Industrieländer mit dem großen Problem der Behandlung von an der Alzheimer-Krankheit erkrankten Personen zu tun, was eine überaus wichtige soziale Aufgabe darstellt. Dabei werden zur Behandlung dieser Erkrankung ausschließlich Amyridin® und Takrin® verwendet.

Wie Untersuchungen ergeben haben, ist die Wirkung der angeführten Präparate auf die Alzheimer-Krankheit jedoch unzureichend. Die Suche nach neuen wirksameren Mitteln zur Be handlung der Alzheimer-Krankheit und anderer Arten von Demenz sowie von Gedächtnisstörungen ist nach wie vor ein aktuelles Problem.

Der Erfindung liegt daher die Aufgabe zugrunde, neue Verbindungen zu entwickeln, die verglichen mit den bekanntten Verbindungen eine erheblich höhere Aktivität aufweisen und die Fähigkeit besitzen Erregungen im Nerven- und Muskelsystem zu stimulieren, das Gedächtnis wiederherzustellen, sowie antiarrhythmische und analgetische Wirkung auszuüben.

Diese Aufgabe wird durch die neuen 4-Amino-2,3-disubst.-6,7-dihydro-5H-1-pyrindinderivate der allgemeinen Formel

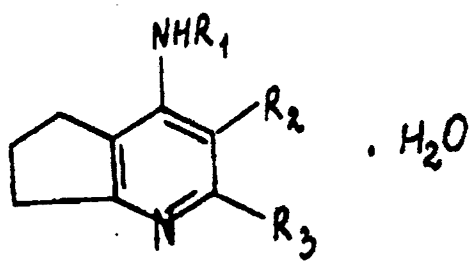

in welcher $R_1$ Wasserstoff oder Niederalkyl,

$R_2$ Niederalkyl oder Phenyl und
$R_3$ Niederalkyl bedeuten,
mit der Ausnahme, daß $R_2$ = $R_3$ = $CH_3$ und $R_1$ Wasserstoff ist,
gelöst.

Die Aufgabe wird ferner durch die 4-Amino-2,3-disubst.-6,7-dihydro-5H-1-pyrindinderivate der allgemeinen Formel

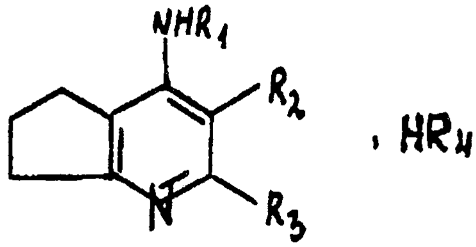

gelöst,
in welcher $R_1$ Wasserstoff bedeutet,
$(R_2 + R_3) = (-CH_2-)_n$, wobei n 3 oder 5 ist und
$HR_4$ HCl oder $HClO_4$ bedeutet,
sowie durch die 4-Amino-2,3-disubst.-6,7-dihydro-5H-1-pyrindinderivate der allgemeinen Formel

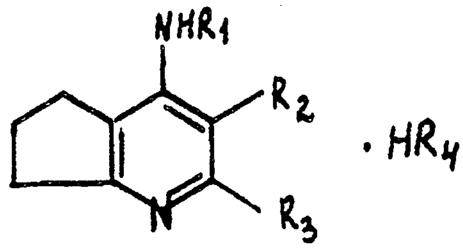

in welcher $R_1$ Wasserstoff,
$R_2$ Niederalkyl oder Phenyl,
$R_3$ Niederalkyl und
$HR_4$ eine Säure, ausgewählt aus der Gruppe der anorganischen und organischen Säuren der aliphatischen oder aromatischen Reihe, bedeutet.

In den 4-Amino-2,3-disubst.-6,7-dihydro-5H-1-pyrindinderivaten stellt $R_1$ zweckmäßigerweise Reste dar, die ausgewählt sind aus der Gruppe $CH_3$- oder $C_2H_5$-.

In den 4-Amino-2,3-disubst.-6,7-dihydro-5H-1-pyrindinderivaten stellt $R_2$ bevorzugt Reste dar, die ausgewählt sind aus der Gruppe $CH_3$, $C_2H_5$, $C_3H_7$,$C_4H_9$ oder $C_6H_5$.

In den 4-Amino-2,3-disubst.-6,7-dihydro-5H-1-pyrindinderivaten stellt $R_2$ zweckmäßigerweise $CH_3$ oder $C_2H_5$ dar.

In den 4-Amino-2,3-disubst.-6,7-dihydro-5H-1-pyrindinderivaten stellt $R_2$ insbesondere $CH_3$ dar.

In den 4-Amino-2,3-disubst.-6,7-dihydro-5H-1-pyrindinderivaten stellt $R_3$ zweckmäßigerweise Reste dar, die ausgewählt sind aus der Gruppe $CH_3$, $C_2H_5$ oder $C_4H_9$.

In den 4-Amino-2,3-disubst.-6,7-dihydro-5H-1-pyrindinderivaten ist $R_3$ vorzugsweise $CH_3$.

In den 4-Amino-2,3-disubst.-6,7-dihydro-5H-1-pyrindinderivaten ist $R_4$ insbesondere der Rest einer anorganischen Säure, welcher die Elemente V - VII der Gruppe 1 - 3 des Periodensystems sowie Br- oder J- umfaßt.

In den 4-Amino-2,3-disubst.-6,7-dihydro-5H-1-pyrindinderivaten ist die bevorzugte anorganische Säure ausgewählt aus der Gruppe HCl, $HClO_4$, HBr, $H_2SO_4$ oder $H_3PO_4$.

In den 4-Amino-2,3-disubst.-6,7-dihydro-5H-1-pyrindinderivaten ist die anorganische Säure vorzugsweise ausgewählt aus den Säuren $HClO_4$ oder $H_2SO_4$.

In den 4-Amino-2,3-disubst.-6,7-dihydro-5H-1-pyrindinderivaten ist die organische aliphatische oder aromatische Säure zweckmäßigerweise eine Monocarbonsäure, ausgewählt aus der Gruppe Essig-, Capron-, Capryl-, Palmin-, Stearin-, Benzoe-, Orthotoluyl- und Nikotinsäure.

In den 4-Amino-2,3-disubst.-6,7-dihydro-5H-1-pyrindinderivaten ist die organische aliphatische oder

aromatische Säure vorzugsweise eine Monocarbonsäure, ausgewählt aus der Gruppe Essig-, Capron-, Capryl- oder Nikotinsäure.

In den 4-Amino-2,3-disubst.-6,7-dihydro-5H-1-pyrindinderivaten ist die organische aliphatische oder aromatische Säure zweckmäßigerweise eine Monocarbonsäure, ausgewählt aus der Gruppe Essig- oder Nikotinsäure.

In den 4-Amino-2,3-disubst.-6,7-dihydro-5H-1-pyrindinderivaten ist die organische aliphatische oder aromatische Säure zweckmäßigerweise eine ungesättigte Säure, ausgewählt aus der Gruppe Malein-, Fumar- oder Zimtsäure.

In den 4-Amino-2,3-disubst.-6,7-dihydro-5H-1-pyrindinderivaten ist die ungesättigte organische Säure vorzugsweise die Zimtsäure.

In den 4-Amino-2,3-disubst.-6,7-dihydro-5H-1-pyrindinderivaten ist die organische aliphatische Säure zweckmäßigerweise eine Dicarbonsäure, ausgewählt aus der Gruppe Oxal-, Malon-, Bernstein- oder Adipinsäure.

In den 4-Amino-2,3-disubst.-6,7-dihydro-5H-1-pyrindinderivaten ist die organische aliphatische Dicarbonsäure vorzugsweise die Oxal- oder Adipinsäure.

In den 4-Amino-2,3-disubst.-6,7-dihydro-5H-1-pyrindinderivaten ist die organische aliphatische Dicarbonsäure insbesondere die Adipinsäure.

In den 4-Amino-2,3-disubst.-6,7-dihydro-5H-1-pyrindinderivaten ist die organische aliphatische oder aromatische Säure zweckmäßigerweise eine Mono-, Di- oder Tricarbonoxisäure, ausgewählt aus der Gruppe, bestehend aus Salicyl-, Wein-oder Zitronensäure.

In den 4-Amino-2,3-disubst.-6,7-dihydro-5H-1-pyrindinderivaten ist die organische aliphatische oder aromatische Oxisäure vorzugsweise die Salicyl- oder Weinsäure.

In den 4-Amino-2,3-disubst.-6,7-dihydro-5H-1-pyrindinderivaten ist die organische aliphatische oder aromatische Oxisäure zweckmäßigerweise die Weinsäure.

In den 4-Amino-2,3-disubst.-6,7-dihydro-5H-1-pyrindinderivaten ist die organische aliphatische oder aromatische Säure zweckmäßigerweise die Glutaminsäure.

Erfindungsgemäß werden somit neue 4-Amino-2,3-disubst.-6,7-dihydro-5H-1-pyrindinderivate, und zwar die bicyclischen Basen, sowie die Salze der tri-und bicyclischen Basen. Niederalkyl bedeuten insbesondere Alkyl mit $C_1$-$C_4$.

Die erfindungsgemäßen Verbindungen werden auf folgende Weise hergestellt.

Die 4-Amino-2,3-disubst.-6,7-dihydro-5H-1-pyrindinderivate der allgemeinen Formel

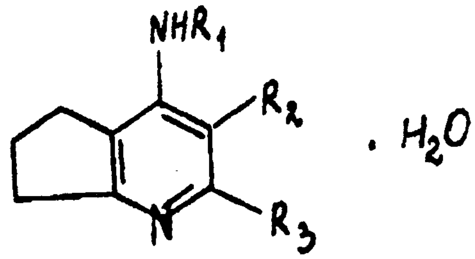

in welcher $R_1$ Wasserstoff oder Niederalkyl,
$R_2$ Niederalkyl oder Phenyl und
$R_3$ Niederalkyl bedeutet,
mit der Ausnahme, daß $R_2 = R_3 = CH_3$ und $R_1$ Wasserstoff ist,
erhält man durch Umsetzung von 2-Aminocyclopentencarbonitril mit einem Keton bei einem Molarverhältnis von 1:1,1-1,3 in Anwesenheit von Polyphosphorsäure, wobei die Reaktion in einem 7- bis 12-fachen Überschuß von Polyphosphorsäure in bezug auf 2-Amino-cyclopentencarbonitril bei 50 bis 140 °C während 2 bis 5 Stunden durchgeführt wird, wonach man das auf 20 bis 80 °C abgekühlte Reaktionsgemisch mit Wasser (1:4-6) verdünnt, drei Mal mit Ether oder Chloroform extrahiert, die organische Schicht abtrennt, die zurückgebliebene wäßrige Schicht mit Alkali bis zu einem pH von 6-7 neutralisiert, den ausgefallenen Niederschlag abfiltriert, das Filtrat mit einer wäßrigen Ammoniaklösung bis zu einem pH von 9-10 behandelt, den erhaltenen Niederschlag der Base abfiltriert und aus einem organischen Lösungsmittel unter Zugabe von Wasser umkristallisiert, wobei bei $R_1$ = Niederalkyl die erhaltene Base zu den Pyridin-4-Chlorderivaten diazotiert wird, die man dann aminoalkyliert.

Die 4-Amino-2,3-disubst.-6,7-dihydro-5H-1-pyrindinderivate der allgemeinen Formel

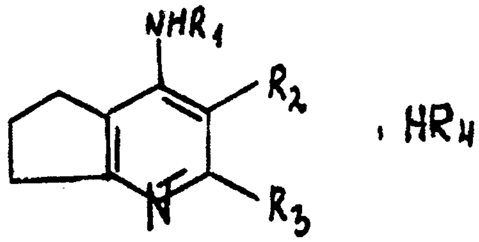

in welcher $R_1$ Wasserstoff bedeutet,
$(R_2 + R_3)$ gleich $(-CH_2-)_n$ ist, wobei n 3 oder 5 ist und
$HR_4$ HCl oder $HClO_4$ bedeutet,
erhält man, indem man 2-Aminocyclopentencarbonitril mit einem Keton im Molverhältnis 1:1,1-1,3 in Gegenwart von Polyphosphorsäure bis zur Bildung einer Base umsetzt und diese mit einer entsprechenden Säure umsetzt, wobei die Reaktion in einem 7- bis 12-fachen Überschuß von Polyphosphorsäure in bezug auf 2-Aminocyclopentencarbonitril bei 50 bis 140 °C während 2 bis 5 Stunden durchgeführt wird, wonach man das auf 20 bis 80 °C abgekühlte Reaktionsgemisch mit Wasser (1:4-6) verdünnt, drei Mal mit Ether oder Chloroform extra hiert, die organische Schicht abtrennt, die zurückgebliebene wäßrige Schicht mit Alkali bis zu einem pH von 6-7 neutralisiert, den ausgefallenen Niederschlag abfiltriert, das Filtrat mit einer wäßrigen Ammoniaklösung bis zu einem pH von 9-10 behandelt, den erhaltenen Niederschlag der Base abfiltriert und aus einem organischen Lösungsmittel unter Zugabe von Wasser umkristallisiert und die auf diese Weise erhaltene Base mit HCl oder $HClO_4$ in an sich bekannter Weise umsetzt.

Die 4-Amino-2,3-disubst.-6,7-dihydro-5H-1-pyrindinderivate der allgemeinen Formel

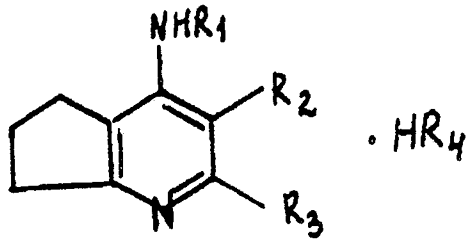

in welcher $R_1$ Wasserstoff,
$R_2$ Niederalkyl oder Phenyl,
$R_3$ Niederalkyl und
$HR_4$ eine Säure, ausgewählt aus der Gruppe der anorganischen und organischen Säuren der aliphatischen oder aromatischen Reihe,
bedeutet, erhält man, indem man 2-Aminocyclopentencarbonitril mit einem Keton im Molverhältnis 1:1,1-1,3 in Anwesenheit von Polyphosphorsäure bis zur Bildung einer Base umsetzt und diese mit einer entsprechenden Säure umsetzt, wobei die Reaktion in einem 7- bis 12-fachen Überschuß von Polyphosphorsäure in bezug auf 2-Aminocyclopentencarbonitril bei 50 bis 140 °C während 2 bis 5 Stunden durchgeführt wird, wonach man das auf 20 bis 80 °C abgekühlte Reaktionsgemisch mit Wasser (1:4-6) verdünnt, drei Mal mit Ether oder Chloroform extrahiert, die organische Schicht abtrennt, die zu rückgebliebene wäßrige Schicht mit Alkali bis zu einem pH von 6-7 neutralisiert, den ausgefallenen Niederschlag abfiltriert, das Filtrat mit einer wäßrigen Ammoniaklösung bis zu einem pH von 9-10 behandelt, den erhaltenen Niederschlag der Base abfiltriert und aus einem organischen Lösungsmittel unter Zugabe von Wasser umkristallisiert und die auf diese Weise erhaltene Base mit einer Säure, ausgewählt aus der Gruppe, bestehend aus anorganischen und organischen aliphatischen oder aromatischen Säuren in an sich bekannter Weise umsetzt.

Die erfindungsgemäßen Verbindungen sind farblose, kristalline Stoffe. Die Salze sind in Wasser gut löslich, die Basen sind in 95 %-igem Alkohol löslich und in Ether und Chloroform unlöslich. Schmelzpunkt: 70 bis 300 °C. Die Struktur der erfindungsgemäßen Verbindungen wird durch die Ergebnisse der Elementaranalyse und der Infrarotspektralanalyse bestätigt.

Es folgt Tabelle 1 mit den erfindungsgemäßen 4-Amino-2,3-disubst.-6,7-dihydro-5H-1-pyrindinderivaten.

Tabelle 1

| Nr. $R_I$ | $R_2$ | $R_3$ | $HR_4$ | Ausbeute % | Schmelzpunkt °C | Bruttoformel | Elementaranalyse, % B - Berechnet, G - Gefunden | C | H | N | Cl, S, P | IR-Spektren kennzeichnende Absorptionsbanden, $cm^{-1}$ Tabletten mit KBr |
|---|---|---|---|---|---|---|---|---|---|---|---|---|
| I | 2 | 3 | 4 | 5 | 6 | 7 | 8 | 9 | I0 | II | I2 | I3 |
| I | H | $CH_3$ | $CH_3$ | HCI Salzsäure | 98 | > 250 (Z.) | $C_{I0}H_{I4}N_2 \cdot HCI$ | B | 56.4 | 7.8 | I2.9 | I6.4 | I628.I652. 3040-3C |
| | | | | | | | | G | 56.4 | 7.7 | I2.4 | I6.6 | 3I95. 3325. 3355 |
| 2 | H | $CH_3$ | $CH_3$ | $HClO_4$ Perchlorsäure | 98 | 203-204 | $C_{I0}H_{I4}N_2 \cdot HClO_4$ | B | 45.7 | 5.7 | I0.6 | I3.5 | I622.I650.3205.32 |
| | | | | | | | | G | 45.8 | 5.9 | I0.5 | I3.4 | 3300.3362.3438 |
| 3 | H | $CH_3$ | $CH_3$ | $H_2SO_4$ Schwefelsäure | 98 | 268-272 | $C_{I0}H_{I4}N_2 \cdot H_2SO_4$ | B | 46.I | 6.2 | I0.8 | I2.3 | I632.I658.3I80. |
| | | | | | | | | G | 46.4 | 6.2 | I0.3 | I2.2 | 3248.3350.3470 |
| 4 | H | $CH_3$ | $CH_3$ | $H_2PO_4$ Phosphorsäure | 97 | > 230 (Z) | $C_{I0}H_{I4}N_2 \cdot H_3PO_4$ | B | 46.2 | 6.6 | I0.8 | II.9 | I505.I635.I660. |
| | | | | | | | | G | 46.8 | 6.8 | I0.5 | | 3200. 3350.3465 |
| 5 | H | $CH_3$ | $CH_3$ | $CH_3COOH$ Essigsäure | 97 | I23-I26 | $C_{I0}H_{I4}N_2 \cdot C_2H_4O_2$ | B | 64.9 | 8.I | I2.6 | | I580.I627.I658. |
| | | | | | | | | G | 64.5 | 9.0 | I2.9 | | 3I75.3300 |
| 6 | H | $CH_3$ | $CH_3$ | $C_6H_5COOH$ Benzoesäure | 96 | 85-88 | $C_{I0}H_{I4}N_2 \cdot C_7H_6O_2$ | B | 67.5 | 7.3 | 9.3 | | I560.I600.I630.I6 |
| | | | | | | | | G | 67.3 | 7.2 | 8.9 | | 3070.3I95.3335-33 |

Tabelle 1 (Fortsetzung)

| 1 | 2 | 3 | 4 | 5 | 6 | 7 | 8 | 9 | 10 | 11 | 12 | 13 |
|---|---|---|---|---|---|---|---|---|---|---|---|---|
| 7 | H | $CH_3$ | $CH_3$ | $C_5H_4NCOOH$<br>Nikotinsäure | 77 | 144-147 | $C_{10}H_{14}N_2 \cdot C_6H_5NO_2 \cdot 2\ H_2O$ | B 59.6<br>G 60.3 | 7.4<br>7.2 | 13.04<br>13.1 | | 1553.1595.1625.<br>1650.3200.3205.<br>3330.3350-3500 |
| 8 | H | $CH_3$ | $CH_3$ | $o\text{-}HOC_6H_4COOH$<br>Salicylsäure | 95 | 213-216 | $C_{17}H_{20}N_2O_3$ | B 68.0<br>G 68.1 | 6.7<br>6.6 | 9.3<br>9.5 | | 1578.1600.1640.<br>3210.3350-3400 |
| 9 | H | $CH_3$ | $CH_3$ | HOOC-COOH<br>Oxalsäure | 62 | 233-235 | $C_{12}H_{16}N_2O_4 \cdot 0.65\ H_2O$ | B 54.6<br>G 54.6 | 7.3<br>7.4 | 10.6<br>11.2 | | 1632.1650.3050.<br>3200.3329.3358 |
| 10 | H | $CH_3$ | $CH_3$ | HOOC-CH(OH)<br>CH(OH)COOH<br>Weinsäure | 94 | 190-193 | $C_{10}H_{14}N_2 \cdot C_4H_6O_6$ | B 53.8<br>G 54.0 | 6.4<br>6.5 | 9.0<br>9.5 | | 1629.1655.1730.<br>3070.3195.3335.3460 |
| 11 | H | $CH_3$ | $CH_3$ | $HOOCCH_2CH_2CH(NH_2)COOH$<br>Glutaminsäure | 53 | 147-149 | $C_{15}H_{23}N_3O_4 \cdot 2\ H_2O$ | B 52.2<br>G 52.6 | 7.8<br>8.0 | 12.7<br>11.6 | | 1605.1628.1651.<br>3200.3322.3360 |
| 12 | H | $CH_3$ | $CH_3$ | Trans-HOOCCH=<br>=CHCOOH<br>Fumarsäure | 96 | 216-220 | $C_{10}H_{14}N_2 \cdot C_4H_4O_4 \cdot H_2O$ | B 56.8<br>G 56.8 | 6.8<br>6.7 | 9.5<br>9.6 | | ..163( 1660.<br>3175.3250.3330 |

Tabelle 1 (Fortsetzung)

| I | 2 | 3 | 4 | 5 | 6 | 7 | 8 | 9 | I0 | II | I2 | I3 |
|---|---|---|---|---|---|---|---|---|---|---|---|---|
| I3 | H | $CH_3$ | $CH_3$ | Cis-HOOCCH=<br>=CHCOOH<br>Maleinsäure | 98 | I7I-I8I | $C_{IO}H_{I4}N_2 \cdot C_4H_4O_4 \cdot$<br>$\cdot H_2O$ | B 56,8<br>G 56,8 | 6,8<br>6,9 | 9,5<br>9,6 | | I590,I630-I660,<br>I686,3265,3420 |
| I4 | H | $CH_3$ | $CH_3$ | $HOOC-CH_2COOH$<br>Malonsäure | 95 | I05-I06 | $C_{IO}H_{I4}N_2 \cdot C_3H_4O_4 \cdot$<br>$\cdot H_2O$ | B 54,9<br>G 54,6 | 7,0<br>7,I | 9,9<br>I0,0 | | I370,I490,I650,<br>3070,3200,3370 |
| I5 | H | $CH_3$ | $CH_3$ | $C_6H_5CH=CHCOOH$<br>Zimtsäure | 90 | 84-87 | $C_{IO}H_{I4}N_2 \cdot C_9H_8O_2 \cdot$<br>$\cdot H_2O$ | B 69,5<br>G 69,4 | 7,3<br>7,4 | 8,5<br>8,4 | | I560,I650,3I80,<br>3340 |
| I6. | H | $CH_3$ | $CH_3$ | $HOOC(CH_2)_4COOH$<br>Adipinsäure | 95 | I50-I55 | $C_{IO}H_{I4}N_2 \cdot C_6H_{IO}O_4$ | B 62,3<br>G 62,4 | 7,8<br>7,7 | 9,I<br>9,0 | | I635,I665,3200,<br>3350 |
| I7. | H | $CH_3$ | $CH_3$ | $CH_3(CH_2)_4COOH$<br>Capronsäure | 89 | II3-II4 | $C_{IO}H_{I4}N_2 \cdot C_7H_{I2}O_2$ | B 64,9<br>G 64,2 | 9,5<br>9,7 | 9,5<br>9,7 | | I625,I648,3I00,<br>3I90,3330-3360 |
| I8. | H | $CH_3$ | $CH_3$ | $HOOCCH_2C(OH)CH_2$<br>$\cdot COOH$<br>COOH<br>Zitronensäure | 70 | I62-I63 | $C_{IO}H_{I4}N_2 \cdot C_6H_8O_7$ | B 54,2<br>G 53,9 | 6,2<br>6,3 | 7,9<br>8,0 | | I285,I380,I470,<br>I590,I750,3I40,<br>3265,3370,3435 |
| I9. | $CH_3$ | $CH_3$ | $CH_3$ | $H_2O$ | 60 | I60-I62 | $C_{II}H_{I6}N_2 \cdot H_2O$ | B 68,0<br>G 67,8 | 9,3<br>9,2 | I4,4<br>I4,6 | | I420,I448,I455,<br>I528,I585,3330 |

Tabelle 1 (Fortsetzung)

| I | 2 | 3 | 4 | 5 | 6 | 7 | 8 | 9 | I0 | II | I2 | I3 |
|---|---|---|---|---|---|---|---|---|---|---|---|---|
| 20 | $C_2H_5$ | $CH_3$ | $CH_3$ | $H_2O$ | 58 | I39-I40 | $C_{I2}H_{I8}N_2 \cdot H_2O$ | B 69.2<br>G 69.4 | 9.6<br>9.7 | I3.5<br>I3.2 | | I420.I445.I525.<br>I582.3280-33I0 |
| 2I | H | $C_2H_5$ | $CH_3$ | $H_2O$ | I0 | I40-I42 | $C_{II}H_{I6}N_2 \cdot H_2O$ | B 68.0<br>G 68.I | 9.3<br>9.3 | I4.4<br>I4.3 | | I580.I642.3200.<br>33I5.3430.3480 |
| 22 | H | $C_2H_5$ | $CH_3$ | $HCI \cdot 2H_2O$ | 85 | II4-II6 | $C_{II}H_{I6}N_2 \cdot HCI \cdot 2H_2O$ | B 53.I<br>G 53.5 | 8.4<br>8.2 | II.2<br>II.2 | I4.3<br>I4.7 | I476.I498.I623.<br>I648.3208.3325.<br>3350 |
| 23 | H | $C_3H_7$ | $CH_3$ | $2H_2O$ | 6 | I56-I58 | $C_{I2}H_{I3}N_2 \cdot 2H_2O$ | B 63.7<br>G 63.4 | 9.7<br>9.5 | I2.4<br>I2.I | | I590.I660.3360.<br>3435 |
| 24 | H | $C_3H_7$ | $CH_3$ | $HCI \cdot 2H_2O$ | 90 | I99-20I | $C_{I2}H_{I8}N_2 \cdot HCI \cdot 2H_2O$ | B 54.8<br>G 55.0 | 8.8<br>8.6 | I0.7<br>II.0 | I3.5<br>I3.9 | I630.I658.3050.<br>3I90.3330.3360 |
| 25. | H | $C_4H_9$ | $CH_3$ | $H_2O$ | 8 | I62-I64 | $C_{I3}H_{20}N_2 \cdot H_2O$ | B 70.3<br>G 70.I | 9.9<br>9.9 | I2.6<br>I2.7 | | I589.I648.3I90.<br>3220.3480 |
| 26 | H | $C_4H_9$ | $CH_3$ | HCI | 92 | 242-246 | $C_{I3}H_{20}N_2 \cdot HCI$ | B 64.9<br>G 64.3 | 8.7<br>8.8 | II.6<br>II.5 | I4.8<br>I4.9 | I480.I500.I625.<br>I655.3200.3370 |

Tabelle 1 (Fortsetzung)

| I | 2 | 3 | 4 | 5 | 6 | 7 | 8 | 9 | I0 | II | I2 | I3 |
|---|---|---|---|---|---|---|---|---|---|---|---|---|
| 27 | H | $CH_3$ | $C_2H_5$ | $H_2O$ | 40 | I47–I50 | $C_{II}H_{I6}N_2 \cdot H_2O$ | B 68.0<br>G 68.0 | 9.3<br>9.4 | I4.4<br>I4.7 | | I582.I665.3230.<br>3378.3420–3470 |
| 28 | H | $CH_3$ | $C_2H_5$ | $HCl.2H_2O$ | 92 | 2I5–2I7 | $C_{II}H_{I6}N_2 \cdot HCl.$<br>$.2H_2O$ | B 53.I<br>G 53.7 | 8.5<br>8.3 | II.3<br>II.3 | I4.3<br>I4.3 | I623.I650.3065.<br>3I95.3325.3370 |
| 29 | H | $CH_3$ | $C_2H_5$ | $HClO_4$ | 84 | I64–I66 | $C_{II}H_{I6}N_2 \cdot HClO_4$ | B 47.7<br>G 47.8 | 6.2<br>6.2 | I0.I<br>9.6 | I2.8<br>I2.7 | I552.I628.I652.<br>3200.3258.3345.<br>3370.3445 |
| 30 | H | $CH_3$ | $C_2H_5$ | $H_2SO_4$ | 95 | I89–I9I | $C_{II}H_{I6}N_2 \cdot H_2SO_4$ | B 48.2<br>G 48.2 | 6.8<br>6.6 | I0.9<br>I0.2 | II.7<br>II.5 | I230.I555.I625.<br>I648.3060.3I95.<br>3327.3360 |
| 3I | H | $CH_3$ | $C_2H_5$ | $H_2SO_4 \cdot H_2O$ | 90 | 243–246 | $C_{22}H_{32}N_4 \cdot H_2SO_4 \cdot$<br>$\cdot H_2O$ | B 56.4<br>G 56.4 | 7.7<br>7.8 | I2.0<br>II.4 | 6.8<br>6.9 | I080.I555.I626.<br>I650.I670.3I95.<br>3320 |
| 32 | H | $C_6H_5$ | $CH_3$ | $H_2O$ | I2 | 204–206 | $C_{I5}H_{I6}N_2 \cdot H_2O$ | B 74.4<br>G 74.5 | 7.4<br>7.6 | II.6<br>II.3 | | I579.I603.I648.<br>3I60.3298.3455 |
| 33 | H | $C_6H_5$ | $CH_3$ | $HCl.H_2O$ | 93 | 252–255 | $C_{I5}H_{I6}N_2 \cdot HCl.$<br>$.H_2O$ | B 64.6<br>G 64.2 | 6.8<br>6.7 | I0.I<br>9.2 | I2.8 | I475.I632.3025.<br>3I55.3280.3458 |
| 34 | H | $CH_3$ | $C_4H_9$ | $H_2O$ | I0 | I2I–I23 | $C_{I3}H_{20}N_2 \cdot H_2O$ | B 70.3<br>G 70.0 | 9.9<br>9.7 | I2.6<br>I2.8 | | I585.I645.3I90.<br>3320.3480 |

Tabelle 1 (Fortsetzung)

| I | 2 | 3 | 4 | 5 | 6 | 7 | 8 | | 9 | 10 | 11 | 12 | 13 |
|---|---|---|---|---|---|---|---|---|---|---|---|---|---|
| 35 | H | $CH_3$ | $C_4H_9$ | $HCl.H_2O$ | 90 | 165-167 | $C_{13}H_{20}N_2.HCl.H_2O$ | B | 60,3 | 8,9 | 10,8 | | 1489,1625,1650, |
| | | | | | | | | G | 59,9 | 9,0 | 10,3 | | 3197,3330 |
| 36 | H | $-(CH_2)_3-$ | | $HCl$ | 96 | > 250 | $C_{11}H_{14}N_2.HCl$ | B | 62,7 | 7,1 | 13,3 | 16,8 | 1480,1628,1631, |
| | | | | | | (Z.) | | G | 62,5 | 7,0 | 13,0 | 16,5 | 3050,3200,3323, |
| | | | | | | | | | | | | | 3368 |
| 37 | H | $-(CH_2)_3-$ | | $HClO_4$ | 95 | 227-230 | $C_{11}H_{14}N_2.HClO_4$ | B | 48,1 | 5,5 | 10,2 | 12,9 | 1090,1118,1150, |
| | | | | | | | | G | 47,8 | 5,8 | 10,0 | 12,6 | 1510,1623,1643, |
| | | | | | | | | | | | | | 3035,3065,3172, |
| | | | | | | | | | | | | | 3327 |
| 38 | H | $-(CH_2)_5-$ | | $HCl$ | 95 | > 250 | $C_{13}H_{18}N_2.HCl$ | B | 65,4 | 8,0 | 11,7 | 14,9 | 1480,1625,1651, |
| | | | | | | (Z.) | | G | 65,3 | 8,2 | 11,3 | 14,7 | 3195,3330 |
| 39 | H | $-(CH_2)_5-$ | | $HClO_4$ | 90 | 213-215 | $C_{13}H_{18}N_2.HClO_4$ | B | 51,6 | 6,3 | 9,3 | 11,7 | 1100,1462,1455, |
| | | | | | | | | G | 51,5 | 6,1 | 9,2 | 11,8 | 1626,1651,3152, |
| | | | | | | | | | | | | | 3198,3260,3369, |
| | | | | | | | | | | | | | 3453 |

40 Vergleichspräparat

H $-(CH_2)_4-HCl.H_2O$ Amiridin

Takrin[R] (THA)

41

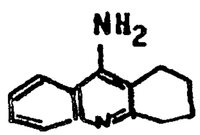

$\cdot\ HCl.H_2O$

In Tierversuchen wurden an isolierten Nerven-Muskelpräparaten, glattmuskulären Organen sowie mit Hilfe des Enzyms Cholinesterase die wichtigsten pharmakologischen Wirkungen der erfindungsgemäßen Verbindungen untersucht.

Ihre akute Toxizität wurde an Mäusen (einmalige intraperitoneale Injektion) nach der Methode der Probit-Analyse nach Zichtfield, Wilcoxon, 1949 untersucht. In Tabelle 2 sind die Parameter der akuten Toxizität ($LD_{50}$) angegeben, die eine mäßige Toxizität der erfindungsgemäßen Verbindungen belegen.

Der Einfluß der erfindungsgemäßen Verbindungen auf das Lernvermögen und das Gedächtnis der Versuchstiere wurde in Versuchen an 20 bis 22 g schweren männlichen Mäusen nach der Methode des "dark avoidance assay" untersucht (s. Elrod K. et al. "An evalutaion of the mechanism of scopolamineinduced impairment in two passive avoidance protocols", Pharmac. Biochem. & Behavior 29, 15-21 /1988/ /Passive avoidance paradigma/). Die Mäuse wurden dabei in den hellen Abschnitt der Kammer gegeben, wonach die Latenzzeit für den Übertritt in die dunkle Kammer, in der die Tiere einem Stromschlag von 0,5 mA ausgesetzt waren, registriert wurde. Danach erhöhte sich die Latenzzeit für den Übertritt in den dunklen Abschnitt und verringerte sich die Aufenthaltsdauer. 30 Minuten vor dem Versuch wurden den Mäusen i.p. 0,7 mg/mg Gedächtnisstörungen hervorrufendes Skopolamin als Cholinblocker injiziert. Die erfindungsgemäßen Verbindungen wurden s.c. 5 Minuten vor dem Lernvorgang injiziert. Das Skopolamin verkürzt die Latenzzeit für den Übertritt in die dunkle Kammer, die erfindungsgemäßen Verbindungen stellen jedoch die Latenzzeitwerte wie bei der Kontrollgruppe wieder her oder bedingen ein geringfügiges Überschreiten dieser Werte (siehe Tabelle 2). Gleichzeitig wird die Aufenthaltsdauer der Tiere im dunklen Abschnitt der Kammer auf 36 bis 45 Hundertstel des Ausgangswerts verringert.

Die bicyclischen Basen (die Verbindungen 19, 20, 21, 23, 25, 27 und 34, ausgenommen 32) zeigten antiamnestische Wirkung, welche die von Amyridin etwas übertraf. Die Salze der tricyclischen Verbindungen (die Stoffe Nr. 36 bis 39) zeigten dabei ausgeprägtere Wirkung. Der maximale Prozentsatz an Tieren, bei denen die durch Skopolamin hervorgerufene Amnesie beseitigt wurde, wurde bei Einwirkung der Salze der bicyclischen Basen, bei denen $R_1 = H$ und $R_2 = R_3 = CH_3$ festgestellt.

Der Einfluß auf die Erregung im peripheren Nervensystem wurde am Hüftnerv von männlichen Ratten untersucht. Der Nerv wurde aus den Ratten unter Nembutalnarkose (60 mg/kg) isoliert. Mit Hilfe von Ag-Makroelektroden wurde der Nerv gereizt, wobei die Reaktionen auf dem Bildschirm des Oszillographen registriert wurden. Die Blockade der Aktionspotentiale (AP) wurde mit dem Lokalanästhetikum Novocain (0,25 %) oder KCl (4 %) ausgelöst. Die erfindungsgemäßen Verbindungen wurden der Perfusionslösung zugesetzt, welche den Nervenbereich in einer Endkonzentration von $1.10^{-5}$ bis $1.10^{-6}$ M umspülte. Alle diese Verbindungen beseitigten die Blockade der Erregung im Nerv und führten zu einer Wiederherstellung der AP-Amplitude zu 40 bis 80 % (s. Tabelle 2), das heißt, was den Wirkungsgrad betrifft, so übertrafen sie Takrin® und erreichten annähernd die Werte von Amyridin.

Der Einfluß der Stoffe auf die Nerven-Muskel-Übertragung wurde am isolierten Nerven-Muskel-Präparat von Mäusen (n.peroneus-m.extensor digitorum longus) (s. Bhattacharyya B. et al. "Neuromuscular blocking action of two hemicholinium-3 analogs" Europ. J. Pharmac. 146, 155-165 /1988/) untersucht, das von Lilly-Lösung perfundiert wurde, durch die man ein Gemisch von $O_2$ und $CO_2$ leitete. Zusammensetzung der Lösung in mM: 135,0 $Na^+$, 4,0 $K^+$, 2,0 $Ca^{2+}$, 1,0 $Mg^{2+}$, 147,8 $Cl^-$, 12,0 $HCO_3^-$, 10,0 $HPO_4^-$, 11,0 Glukose; t - 37° C. Das Endplattenpotential (EPP) und das Endplattenminiaturpotential (minEPP) wurden mit Hilfe eines Magnetografen aufgezeichnet und danach mit dem Computer ausgewertet. Ermittelt wurden die Frequenz (f), die Amplitude (A) und die Dauer (t) der minEPP und der EPP (s. Tabelle 2).

**Tabelle 2**

**Akute Toxizität, Nebenwirkung und wichtigste pharmakologische Wirkungen der erfindungsgemäßen Verbindungen**

| # der Verbindung (gemäß Tab. 1) | $LD_{50}$ mg/kg i.p. | $TD_{50}$ x) mg/kg (Tremor) | Verhältnis $TD_{50}$ zu $ED_{50}$ | Einfluß auf das Gedächtnis: Zahl der Tiere in %, bei denen die Skopolaminwirkung aufgehoben wurde | Wiederherstellung des AP im Hüftnerv d. Ratte, in % (nach KCl-Blockade) | Stimulierung der Nerven-Muskel-übertragung, in % der Amplitude EPP xxx | minEPP |
|---|---|---|---|---|---|---|---|
| 1 | 2 | 3 | 4 | 5 | 6 | 7 | 8 |
| 1 | 15 | 6 | 15 | 70 | 80 | 50 | 90 |
| 2 | 15 | 5 | 12,5 | 75 | 80 | 45 | 85 |
| 3 | 17 | 7 | 20 | 80 | 87 | 55 | 110 |
| 4 | 15 | 5,5 | 11 | 70 | 74 | 40 | 85 |
| 5 | 25 | 9 | 13 | 60 | 60 | 40 | 90 |
| 6 | 35 | 10 | 10 | 60 | 75 | 35 | 80 |
| 7 | 20 | 9 | 9 | 80 | 70 | 52 | 95 |
| 8 | 17 | 8 | 6 | 70 | 78 | 40 | 75 |
| 9 | 15 | 7 | 7 | 70 | 65 | 40 | 80 |
| 10 | 15 | 6 | 7,5 | 80 | 75 | 38 | 79 |
| 11 | 17 | 10 | 14 | 75 | 82 | 40 | 85 |
| 12 | 15 | 7 | 8 | 50 | 65 | 32 | 70 |
| 13 | 18 | 8 | 8,5 | 65 | 70 | 35 | 72 |

x $TD_{50}$ = toxische Dosis, die bei 50 % der Tiere einen Tremor bewirkt

xx $ED_{50}$ = wirksame Dosis, die bei 50 % der Tiere die Wirkung von Skopolamin blockiert

xxx EPP = Endplattenpotential

Tabelle 2 (Fortsetzung)

| 1 | 2 | 3 | 4 | 5 | 6 | 7 | 8 |
|---|---|---|---|---|---|---|---|
| 14 | 17 | 9 | 8,2 | 70 | 75 | 30 | 62 |
| 15 | 16 | 7 | 6,8 | 72 | 60 | 40 | 75 |
| 16 | 21 | 10 | 6,6 | 75 | 70 | 42 | 78 |
| 17 | 24 | 10 | 7,1 | 72 | 75 | 43 | 75 |
| 18 | 16 | 9 | 6 | 70 | 68 | 45 | 80 |
| 19 | 16 | 12 | 4 | 70 | 45 | 28 | 56 |
| 20 | 18 | 10 | 3,8 | 65 | 40 | 30 | 58 |
| 21 | 30 | 10 | 3,8 | 68 | 60 | 25 | 58 |
| 22 | 32 | 12 | 4 | 70 | 55 | 25 | 60 |
| 23 | 28 | 9 | 3,2 | 65 | 50 | 20 | 38 |
| 24 | 30 | 10 | 3 | 68 | 45 | 25 | 54 |
| 25 | 25 | 13 | 3,5 | 65 | 60 | 22 | 46 |
| 26 | 26 | 11 | 4 | 70 | 45 | 20 | 45 |
| 27 | 30 | 19 | 10 | 60 | 65 | 23 | 45 |
| 28 | 40 | 20 | 11 | 80 | 82 | 22 | 48 |
| 29 | 35 | 22 | 12 | 70 | 60 | 18 | 40 |
| 30 | 40 | 22 | 11 | 75 | 75 | 24 | 45 |
| 31 | 40 | 23 | 12 | 70 | 80 | 28 | 50 |
| 32 | 18 | 10 | 2,6 | 50 | 35 | 20 | 45 |
| 33 | 25 | 15 | 2,4 | 55 | 40 | 20 | 42 |
| 34 | 15 | 5 | 1,8 | 65 | 38 | 18 | 38 |
| 35 | 17 | 6 | 2,2 | 68 | 40 | 18 | 40 |
| 36 | 36 | 15 | 7,5 | 65 | 50 | 20 | 60 |
| 37 | 38 | 18 | 8,2 | 68 | 45 | 30 | 66 |
| 38 | 49 | 17 | 7,6 | 70 | 40 | 28 | 52 |
| 39 | 50 | 18 | 7,2 | 72 | 40 | 25 | 60 |
| | | | | Vergleichspräparate | | | |
| 40 | 44 | 23,2 | 16 | 60 | 80 | 55 | 100 |
| 41 | 30 | 10 | 5 | 15 | 32 | 22 | 40 |

Sämtliche erfindungsgemäßen Verbindungen zeigten sowohl eine Wirkung auf das minEPP als auch auf das EPP, wobei sie zu einer Steigerung der Amplitude und der Dauer führten; die Frequenz blieb unverändert. Im Gegensatz dazu beeinflussen die klassischen Cholinesteraseinhibitoren lediglich das minEPP, indem sie ihre Amplitude und die Dauer steigern. Die Fähigkeit der erfindungsgemäßen Verbindungen, die Amplitude und die Dauer des EPP zu steigern und "Riesen"-EPP's hervorzurufen, zeigt, daß sie präsynaptisch wirksam sind und eine Steigerung des Transmitterausstoßes bewirken. Diese Wirkung war bei den Salzen der bicyclischen Basen am stärksten ausgeprägt und entspricht dem Wirkungsgrad von Amyridin.

In Versuchen an glattmuskulären Organen (Uterus, Samenleiter der Ratte und Krummdarm des Meerschweinchens) wurden die Wirkung der erfindungsgemäßen Verbindungen auf die kontraktile Aktivität

der glatten Muskeln und ihre Reaktionen auf den Einfluß von Acetylcholin, Serotonin, Adrenalin und KCl als Agonisten untersucht. Mit Hilfe eines Schreibers wurden die Amplitude und die Dauer der Kontraktion der glatten Muskeln unter Verwendung eines Geräts vom Typ Mechanstron-untersucht.

Die Salze der bicyclischen und tricyclischen Basen steigerten am stärksten die kontraktilen Reaktionen der Muskeln auf Adrenalin und Serotonin.

Im Hinblick auf die Reaktion auf Acetylcholin unterschieden sich die Salze der bicyclischen Basen von anderen Verbindungen. Sie rufen eine starke und langanhaltende, jedoch sich erst allmählich einstellende spontane Muskelkontraktion des Krummdarms hervor, beeinflußen jedoch nicht die kontraktile Reaktion des Muskels auf Acetylcholin.

Die Salze der bicyclischen Basen rufen in allen Konzentra tionen eine spontanere Kontraktion sämtlicher glattmuskulärer Objekte hervor als Amyridin und verstärken die kontraktilen Reaktionen der Muskel auf sämtliche Agonisten mit Ausnahme von KCl (s. Tabelle 3). Sie induzieren nicht nur eine spontane Kontraktion des Uterus, sondern im Gegensatz zu Takrin auch eine solche des Samenleiters und des Darms.

Die erzielten Ergebnisse zeigen, daß die Salze der bicyclischen Basen eine neue Wirkung aufweisen und zwar einen M-cholinomimetischen Effekt. Durch den unmittelbaren Einfluß auf die M-Cholinrezeptoren des Darms erklärt sich auch ihr Vermögen, eine starke spontane Kontraktion des Muskels auszulösen. Daß dabei sich die Wirkung des Acetylcholins nicht ändert, hängt mit der konkurrierenden Abbindung der Verbindungen durch die vorliegende Gruppe von Rezeptoren zusammen, weshalb das Acetylcholin nicht seine stimulierende Wirkung ausüben kann (s. Tabelle 3). Diese Tatsache ist von besonderer Bedeutung auch für ihren stärkeren Einfluß auf das Gedächtnis der Versuchstiere.

Tabelle 3

EINFLUSS DER ERFINDUNGSGEMÄSSEN VERBINDUNGEN AUF DIE KONTRAKTILE AKTIVITÄT VON GLATTMUSKELOBJEKTEN

| Verbindung Nr. | Konzentration (Mol) n = 9 | Objekt – Agonist: Uterus der Ratte | | | | | | Samenleiter der Ratte | | |
|---|---|---|---|---|---|---|---|---|---|---|
| | | Spontane Kontraktion in %, bezogen auf die Wirkung von Serotonin | Serotonin $1.10^{-6}$ (Mol): Amplitude, % bezogen auf die Kontrolle | Serotonin $1.10^{-6}$ (Mol): Dauer, Min. | Spontane Kontraktion in %, bezogen auf die Wirkung von Acetylcholin | Acetylcholin $1.10^{-6}$ (Mol): Amplidude, % bezogen auf d. Kontrolle | Acetylcholin $1.10^{-6}$ (Mol): Dauer, Min. | Spontane Kontraktion in %, bezogen auf die Wirkung von Adrenalin | Adrenalin $1.10^{-6}$: Amplitude, % bezogen auf d. Kontrolle | Adrenalin $1.10^{-6}$: Dauer, Min. |
| I | 2 | 3 | 4 | 5 | 6 | 7 | 8 | 9 | I0 | II |
| I | $I.I0^{-5}$ | 220 | I50 | 25±0.4 | 75 | unverändert | 2±0.05 | 40 | I65 | 28±0.2 |
| 2 | $I.I0^{-5}$ | 200 | I40 | 25±0.8 | 65 | -"- | 2.2±0.05 | 35 | I45 | 28 |
| 3 | $I.I0^{-5}$ | I80 | I45 | 24±0.6 | 60 | -"- | 2.5±0.04 | 30 | I40 | 25±0.8 |
| 4 | $I.I0^{-5}$ | I50 | I55 | 26±0.4 | 58 | -"- | 2.2±0.05 | 38 | I45 | 28±0.6 |
| 5 | $I.I0^{-5}$ | I82 | I60 | 22±0.2 | 49 | -"- | 2.5±0.08 | 35 | I48 | 24±0.4 |
| 6 | $I.I0^{-5}$ | I40 | I50 | 25±0.3 | 42 | -"- | 2.I±0.06 | 45 | I58 | 24±0.5 |
| 7 | $I.I0^{-5}$ | 200 | I60 | 26±0.4 | 50 | -"- | 2.5±0.05 | 48 | I50 | 24±0.06 |
| 8 | $I.I0^{-5}$ | I60 | I65 | 22±0.5 | 52 | -"- | 2.5±0.05 | 48 | I50 | 24±0.06 |

Tabelle 3 (Fortsetzung)

| I | 2 | 3 | 4 | 5 | 6 | 7 | 8 | 9 | I0 | II |
|---|---|---|---|---|---|---|---|---|---|---|
| 9 | $I.I0^{-5}$ | I45 | I60 | 26±0,4 | 50 | unverändert | 2,8±0,02 | 40 | I45 | 22±0,06 |
| I0 | $I.I0^{-4}$ | 300 | I60 | 32±0,8 | 68 | -"- | 2,5±0,05 | 52 | I60 | 26±0,08 |
| II | $I.I0^{-4}$ | 290 | I70 | 26±0,6 | 65 | -"- | 2,8±0,08 | 40 | I52 | 20±0,06 |
| I2 | $I.I0^{-5}$ | 200 | I48 | 22±0,5 | 45 | -"- | 2,2±0,08 | 45 | I48 | 20±0,4 |
| I3 | $I.I0^{-5}$ | I50 | I60 | 25±0,8 | 40 | -"- | 2,5±0,06 | 50 | I50 | 22±0,8 |
| I4 | $I.I0^{-5}$ | I50 | I62 | 24±0,6 | 45 | -"- | 2,3±0,08 | 40 | I48 | 20±0,6 |
| I5 | $I.I0^{-5}$ | I50 | I38 | 20±0,5 | 46 | -"- | 2,5±0,06 | 40 | I45 | 22±0,5 |
| I6 | $I.I0^{-5}$ | I80 | I75 | 25±0,6 | 50 | -"- | 2,3±0,08 | 58 | I50 | 24±0,6 |
| I7 | $I.I0^{-5}$ | I60 | I80 | 28±0,7 | 60 | -"- | I,3±0,06 | 50 | I48 | 25±0,6 |
| I8 | $I.I0^{-5}$ | I58 | I75 | 28±0,6 | 58 | -"- | I,5±0,05 | 45 | I40 | 24±0,6 |
| I9 | $I.I0^{-4}$ | 80 | I20 | I4±0,5 | 25 | -"- | I,2±0,06 | 22 | II5 | I2 |
| 20 | $I.I0^{-4}$ | 75 | I25 | I5±0,4 | 28 | -"- | I,8±0,05 | 25 | I20 | I2±0,02 |
| 2I | $I.I0^{-4}$ | 70 | II8 | I0±0,5 | 22 | -"- | 0,5±0,05 | 25 | II5 | I0 |
| 22 | $I.I0^{-4}$ | 65 | I22 | I2±0,5 | 24 | -"- | 0,8±0,06 | 28 | I20 | I0 |
| 23 | $I.I0^{-4}$ | 58 | I20 | I2±0,8 | 25 | -"- | 0,5±0,04 | 25 | II5 | I0 |

Tabelle 3 (Fortsetzung)

| I | 2 | 3 | 4 | 5 | 6 | 7 | 8 | 9 | I0 | II |
|---|---|---|---|---|---|---|---|---|---|---|
| 24 | $I.I0^{-4}$ | 55 | II9 | I5±0,6 | 28 | unverändert | 0,6±0,05 | 28 | I20 | I0±0,5 |
| 25 | $I.I0^{-4}$ | 45 | I28 | 20±0,8 | 25 | -"- | 0,8±0,02 | 26 | I20 | I5±0,6 |
| 26 | $I.I0^{-4}$ | 48 | I28 | 22±0,6 | 28 | -"- | 0,5±0,05 | 30 | I25 | I6±0,2 |
| 27 | $I.I0^{-4}$ | 45 | I25 | 20±0,4 | 30 | -"- | 0,9±0,02 | 30 | I26 | I5±0,4 |
| 28 | $I.I0^{-4}$ | 46 | I30 | 2I±0,2 | 32 | -"- | I,2±0,06 | 32 | I30 | I5±0,2 |
| 29 | $I.I0^{-4}$ | 52 | I35 | 2I±0,4 | 30 | -"- | 0,8±0,05 | 30 | I25 | I4±0,2 |
| 30 | $I.I0^{-4}$ | 50 | I35 | 20±0,5 | 28 | -"- | 0,6±0,02 | 35 | II8 | I5±0,4 |
| 3I | $I.I0^{-4}$ | 60 | I40 | 22±0,5 | 25 | -"- | 0,8±0,04 | 38 | I28 | I5±0,5 |
| 32 | $I.I0^{-4}$ | 50 | I35 | 20±0,5 | 25 | -"- | 0,9±0,05 | 40 | I30 | I5±0,6 |
| 33 | $I.I0^{-4}$ | 45 | I40 | 20±0,5 | 32 | -"- | 0,6±0,05 | 42 | I28 | I5±0,5 |
| 34 | $I.I0^{-4}$ | 40 | I28 | 22±0,8 | 35 | -"- | 0,9±0,02 | 45 | I25 | I2±0,2 |
| 35 | $I.I0^{-4}$ | 45 | I45 | 20±0,6 | 30 | -"- | 0,6±0,05 | 40 | I20 | I5±0,7 |
| 36 | $I.I0^{-5}$ | I80 | I85 | 26±0,3 | 22 | I40 | I5±0,4 | 50 | I60 | I3±0,4 |
| 37 | $I.I0^{-5}$ | I60 | I45 | 25±0,6 | 25 | I35 | I8±0,5 | 40 | I48 | I5±0,2 |

Tabelle 3 (Fortsetzung)

| I | 2 | 3 | 4 | 5 | 6 | 7 | 8 | 9 | I0 | II |
|---|---|---|---|---|---|---|---|---|---|---|
| 38 | $I.IO^{-5}$ | 200 | I80 | 25±0.8 | 28 | I25 | 20±0.6 | 40 | I45 | I6±0.2 |
| 39 | $I.IO^{-5}$ | 2I0 | I65 | 28±0.5 | 25 | I40 | 22±0.6 | 40 | I48 | I8±0.5 |
| Vergleichspräparate | | | | | | | | | | |
| 40 | $I.IO^{-4}$ | 367 | I75 | 30±0.5 | 50 | I95 | 30±0.9 | 60 | I60 | 28±0.8 |
| 40 | $I.IO^{-5}$ | 200 | I45 | 25±0.4 | 30 | I65 | 30±0.5 | 50 | I50 | 25±0.6 |
| 4I | $I.IO^{-4}$ | 200 | 0 | I0 | 0 | 26 | I±0.2 | 0 | 35 | 2±0.2 |
| 4I | $I.IO^{-5}$ | I28 | I64 | 5±0.5 | 0 | I00 | I±0.02 | 0 | I20 | 2±0.06 |

Die antiarrhythmische Wirkung der erfindungsgemäßen Verbindungen wurde in zwei Versuchsserien untersucht, und zwar an isolierten Präparaten des rechten Vorhofs von Kaninchen und mit Hilfe des Calciumchlorid-Arrhythmie-Modells an Ratten. In den Versuchen an den isolierten Präparaten des rechten Vorhofs wurden mit Hilfe von Mikroelektroden die Dauer des AP sowie der Refraktärzeit (R) und die maximale Hemmung ($\theta$) und der Parameter ($\theta$/R) untersucht, durch den die Anfälligkeit des Myokards

gegenüber der Entstehung von Arrhythmien gekennzeichnet wird. In den Versuchen an Ratten wurden diesen Calciumchlorid in einer Dosierung von 250 mg/kg injiziert. Registriert wurden das Vorliegen von Kammerflimmern und die Lebensdauer der Tiere. Sämtliche erfindungsgemäßen Verbindungen üben eine mäßige antiarrhythmische Wirkung aus. Sie steigern die Refraktärzeit des Muskels und des AP um 35 bis 145 % und verringern gleichzeitig die Anfälligkeit des Myokards, ausgedrückt durch den Parameter $\theta$/R um 10 bis 80 % (s. Tabelle 4). In bezug auf diese Wirkung waren die Salze der bicyclischen Basen wirksamer als Amyridin. Bezüglich des Vermögens, die Tiere vor Kammerflimmern und vor dem Tod nach Verabreichung von 250 mg/kg Calciumchlorid zu schützen, waren diese Verbindungen ebenfalls am stärksten wirksam. Bei Verwendung des Calciumchlorid-Arrhythmie-Modells waren viele erfindungsgemäßen Verbindungen stärker wirksam als das klassische Antiarrhythmikum Chinidin (s. M.D. Mašhkovskij, Lekarstvennye sredstva, Bd. 1, S. 401, Moskau, Medicina, 1984).

Bei anderen Modellen wurde dies nicht beobachtet. Die erzielten Ergebnisse zeigen die besondere Wirksamkeit der erfindungsgemäßen Verbindungen bei Arrhythmien, die nach dem Reentry-Mechanismus entstehen.

## Tabelle 4

Antiarrhythmische und analgetische Wirkung der erfindungsgemäßen Verbindungen, sowie ihr Einfluß auf die Orientierungsaktivität und die Wirkung von Hexenal bei Mäusen

| Verbindung Nr. (nach Tab. 1) | Antiarrhythmische Wirkung: Verlängerung d. Refraktärzeit (R), in % | Antiarrhythmische Wirkung: Abnahme d. Parameters θ/R, in % | Verlängerung d. Lebensdauer von Ratten an Hand des $CaCl_2$-Arrhythmie-Modells,[x] in % | Analgetische Wirkung: Abnahme d. Zahl der "Krämpfe" bei Mäusen, in % | Änderung der Orientierungsaktivität, in % (xx) | Änderung der Dauer der Seitenlage, in %: Hexenal 60 mg/kg (x) |
|---|---|---|---|---|---|---|
| 1 | 2 | 3 | 4 | 5 | 6 | 7 |
| 1 | 120 | 70 | 150 | 60 | -25 | +28 |
| 2 | 130 | 80 | 160 | 70 | -15 | +35 |
| 3 | 115 | 65 | 190 | 90 | -8 | 0 |
| 4 | 90 | 55 | 180 | 80 | 0 | 0 |
| 5 | 110 | 60 | 130 | 90 | -10 | 0 |
| 6 | 100 | 50 | 130 | 70 | -20 | +20 |
| 7 | 110 | 62 | 150 | 60 | -25 | +32 |
| 8 | 100 | 58 | 190 | 70 | 0 | 0 |
| 9 | 110 | 60 | 140 | 70 | -20 | +30 |
| 10 | 120 | 68 | 180 | 60 | 0 | 0 |
| 11 | 130 | 75 | 160 | 70 | -10 | +28 |
| 12 | 110 | 50 | 130 | 90 | -15 | +30 |
| 13 | 120 | 62 | 100 | 60 | -10 | +25 |
| 14 | 100 | 58 | 90 | 50 | -7 | +15 |
| 15 | 90 | 40 | 110 | 65 | -15 | +30 |
| 16 | 80 | 30 | 70 | 50 | -10 | +22 |

Tabelle 4 (Fortsetzung)

| 1 | 2 | 3 | 4 | 5 | 6 | 7 |
|---|---|---|---|---|---|---|
| 17 | 80 | 47 | 110 | 80 | 0 | 0 |
| 18 | 110 | 55 | 120 | 90 | -15 | +15 |
| 19 | 50 | 20 | 50 | 30 | 0 | 0 |
| | | | Vergleichspräparate | | | |
| 40 | 119 | 69 | 150 | 30 | +45 | -25 |
| 41 | 75 | 35 | 50 | 45 | -28 | +20 |

1) x = In diesen Tests wurde die Dosierung von 1/10 der $LD_{50}$-Dosis getestet.

2) In der Tabelle sind die typischesten Vertreter der Gruppe der Verbindungen angegeben.

Die analgetische Wirkung der erfindungsgemäßen Verbindungen wurde an männlichen Mäusen anhand des "Krampf"-Tests (Writhing-Tests) unter intraperitonealer Injizierung von Essigsäure und des Hot-plate-Tests untersucht. Im ersten Falle wurde die analgetische Wirkung der Verbindungen anhand der prozentualen Verringerung der Zahl der Krämpfe ermittelt. Beim zweiten Test wurde die Schwelle der Schmerzempfindlichkeit (Ablecken der Pfoten) bestimmt. Die stärkste analgetische Wirkung der erfindungsgemäßen Verbindungen zeigte sich beim "Krampf"-Test. Dabei übertrafen die Salze der bicyclischen Basen Amyridin, aber nicht alle erfindungsgemäßen Verbindungen waren schwächer als Morphin (s. Tabelle 4).

Von Interesse ist der Einfluß der erfindungsgemäßen Verbindungen auf das ZNS und das Verhalten der Tiere. Alle Verbindungen zeigen eine Kombination von stimulierenden und sedativen Eigenschaften und bei höheren Dosen auch eine m-cholinomimetische Wirkung, wobei Tremor, Speichelfluß und Diarrhoe ausgelöst werden.

Die Salze der bicyclischen Basen bewirken den stärksten sedativen Effekt und zusätzlich noch einen m-cholinomimetischen Effekt (s. Tabelle 2). Deshalb zeigen sie einen hohen Kennwert für die Anwendungssicherheit bzw. einen hohen Schutzindex $TD_{50}/ED_{50}$ aufgrund des niedrigen $ED_{50}$-Wertes, wie der Passiv-Avoidance-Test an Mäusen zur Feststellung des Skopolaminantagonismus zeigt.

Untersucht wurden die Wirkmechanismen der erfindungsgemäßen Verbindungen:

1. Die Wirkung auf Acetylcholinesterase menschlicher Erythrozyten auf spektrofotometrischem Wege.
2. Die Wirkung auf die $K^+$- und $Na^+$-Durchlässigkeit der Membrane des Ranvier-Schnürringes (s. Fox T.M. et al. "Modification of ionic membrane currrents of Ranvier nodes by UV-radiation under voltage clamp conditions", Experientia, 27, 1289-1290 /1971/ und Arhom P. et al. "Local anesthetics: effects on permeability properties of nodal membrane in myelynated nerve fibres from xenopus". Acta physiol. Scand. 91, 11-21 /1974/) der Nervenfasern des Hüftnervs des Frosches unter Anwendung der Methode der Messung der Membranspannung (Voltage-clampmethode) (s. Derksen H.E. "Axon membrane voltage fluctuations", Acta Physiol., Pharmacol Neer, 13, 373-466 /1965/).

Alle erfindungsgemäßen Verbindungen sind wirksame Cholinesteraseinhibitoren. Ihre Inhibierungskonstanten IK betragen $4{,}8.10^{-5}$ - $4{,}8.10^{-8}$ Mol (s. Tabelle 5).

## Tabelle 5

Wirkmechanismen der erfindungsgemäßen Verbindungen

| Substanz Nr. | Acetylcholinesteraseinhibierung bei menschlichen Erythrozyten IK, (Mol) | Blockierung der $K^+$-Durchlässigkeit der Membrane $Ic_{50}^x$ (Mol) | Blockierung der $Na^+$-Durchlässigkeit der Membrane $Ic_{50}$ (Mol) |
|---|---|---|---|
| 1 | 2 | 3 | 4 |
| 1 | $10,7.10^{-7}$ | $3,0.10^{-4}$ | $3,5.10^{-4}$ |
| 2 | $4,8.10^{-8}$ | $0,6.10^{-4}$ | $1,0.10^{-4}$ |
| 3 | $1,8.10^{-7}$ | $0,5.10^{-4}$ | $1,5.10^{-4}$ |
| 4 | $2,4.10^{-7}$ | $2,0.10^{-4}$ | $3.10^{-4}$ |
| 5 | $6,6.10^{-7}$ | $2,8.10^{-4}$ | $3,8.10^{-4}$ |
| 6 | $4,0.10^{-7}$ | $2,8.10^{-4}$ | $3,5.10^{-4}$ |
| 7 | $3,66.10^{-7}$ | $2,0.10^{-4}$ | $1,5.10^{-4}$ |
| 8 | $1,59.10^{-7}$ | $2,0.10^{-4}$ | $2,8.10^{-4}$ |
| 9 | $2,51.10^{-7}$ | $2,5.10^{-4}$ | $1,8.10^{-4}$ |
| 10 | $1,23.10^{-7}$ | $0,8.10^{-4}$ | $2,5.10^{-4}$ |
| 11 | $0,8.10^{-7}$ | $1,2.10^{-4}$ | $1,5.10^{-4}$ |
| 12 | $5,2.10^{-7}$ | $1,5.10^{-4}$ | $3,8.10^{-4}$ |
| 13 | $4,5.10^{-7}$ | $2,9.10^{-4}$ | $4,5.10^{-4}$ |
| 14 | $3,5.10^{-7}$ | $3,5.10^{-4}$ | $1,8.10^{-4}$ |
| 15 | $6,5.10^{-7}$ | $4,2.10^{-4}$ | $5,2.10^{-4}$ |
| 16 | $5,8.10^{-7}$ | $3,8.10^{-4}$ | $1,0.10^{-4}$ |
| 17 | $3,9.10^{-7}$ | $4,0.10^{-4}$ | $4,5.10^{-4}$ |
| 18 | $1,45.10^{-7}$ | $4,2.10^{-4}$ | $3,9.10^{-4}$ |
| 19 | $6,7.10^{-6}$ | $1,8.10^{-3}$ | $2,5.10^{-3}$ |
| 20 | $7,0.10^{-6}$ | $2,2.10^{-3}$ | $2,8.10^{-3}$ |
| 21 | $7,0.10^{-6}$ | $3,5.10^{-3}$ | $3,2.10^{-3}$ |
| 22 | $7,0.10^{-6}$ | $3,0.10^{-3}$ | $3,6.10^{-3}$ |
| 23 | $8,0.10^{-6}$ | $4,2.10^{-3}$ | $4,0.10^{-3}$ |
| 24 | $7,5.10^{-6}$ | $4,0.10^{-3}$ | $4,2.10^{-3}$ |
| 25 | $2,8.10^{-8}$ | $8,2.10^{-3}$ | $7,8.10^{-3}$ |

Tabelle 5 (Fortsetzung)

| 1 | 2 | 3 | 4 |
|---|---|---|---|
| 26 | $1{,}5.10^{-5}$ | $8{,}0.10^{-3}$ | $8{,}5.10^{-3}$ |
| 27 | $3{,}8.10^{-6}$ | $1{,}5.10^{-4}$ | $3{,}8.10^{-4}$ |
| 28 | $6{,}1.10^{-6}$ | $1{,}2.10^{-4}$ | $1{,}5.10^{-4}$ |
| 29 | $6{,}0.10^{-6}$ | $2{,}0.10^{-4}$ | $3{,}0.10^{-4}$ |
| 30 | $5{,}1.10^{-6}$ | $2{,}8.10^{-4}$ | $3{,}5.10^{-4}$ |
| 31 | $4{,}7.10^{-6}$ | $3{,}0.10^{-4}$ | $3{,}3.10^{-4}$ |
| 32 | $5{,}0.10^{-5}$ | $6{,}8.10^{-3}$ | $6{,}0.10^{-3}$ |
| 33 | $4{,}8.10^{-5}$ | $6{,}5.10^{-3}$ | $5{,}5.10^{-3}$ |
| 34 | $6{,}7.10^{-6}$ | | |
| 35 | $6{,}5.10^{-6}$ | | |
| 36 | $1{,}1.10^{-7}$ | $4{,}2.10^{-4}$ | $5{,}2.10^{-3}$ |
| 37 | $1{,}5.10^{-7}$ | $4{,}5.10^{-4}$ | $4{,}2.10^{-3}$ |
| 38 | $4{,}8.10^{-7}$ | $5{,}8.10^{-4}$ | $4{,}5.10^{-3}$ |
| 39 | $5{,}6.10^{-7}$ | $4{,}8.10^{-4}$ | $5{,}0.10^{-3}$ |
| Vergleichspräparate | | | |
| 40 | $1{,}47.10^{-7}$ | $4{,}5.10^{-4}$ | $2{,}0.10^{-4}$ |
| 41 | $3{,}61.10^{-7}$ | $8{,}.10^{-4}$ | $1{,}5.10^{-5}$ |

x) Ic = Konzentration, die eine 50 %-ige Blockade der Ionendurchlässigkeit bewirkt

Alle erfindungsgemäßen Verbindungen sind Blocker für die $K^+$-Durchlässigkeit der Membrane analog dem Amyridin. Die Salze der bicyclischen Basen blockieren jedoch stärker die $Na^+$-Durchlässigkeit der Membrane, was sich mit ihren sedativen und analgetischen Eigenschaften in Verbindung bringen läßt.

Eine $\alpha_2$-adrenoblockierende Wirkung zeigt die erfindungsgemäße Verbindung I in demselben Maße wie auch Takrin®, sie fehlt jedoch praktisch bei Amyridin. Dies beweisen spezielle Radioligandenuntersuchungen an einzelnen erfindungsgemäßen Verbindungen (s. Tabelle 6).

Die dabei erzielten Ergebnisse bestätigen den Unterschied im Wirkmechanismus zwischen Amyridin und den erfindungsgemäßen Verbindungen.

## Tabelle 6

**Wechselwirkung einiger erfindungsgemäßer Verbindungen mit den Rezeptoren**

| Stoff | Konzentration, Mol | Verdrängung des 3H-Clonidins in der Hirnrinde des Kaninchens ($\alpha_2$-Adrenorezeptoren) | Verdrängung des 3H-Chinuklidins im Kaninchenherzen ($M_2$-Cholinrezeptoren) | Verdrängung des 3H-Propyl-2-aminobenzylats im Gehirn des Kaninchens (M-cholinrezeptoren) |
|---|---|---|---|---|
| 1 | $1.10^{-6}$ | 0 | 0 | 0 |
| | $1.10^{-5}$ | 26 | 0 | 28 |
| 40 | $1.10^{-6}$ | 0 | 0 | 0 |
| | $1.10^{-5}$ | 10 | 0 | 10 |
| 41 | $1.10^{-6}$ | 0 | 10 | 0 |
| | $1.10^{-5}$ | 27 | 50 | 0 |

Die angeführten Versuchsergebnisse zeigen somit, daß die erfindungsgemäßen Verbindungen einen neuen Typ von pharmakologischer Wirkung aufweisen. Sie besitzen folgende obligatorische Kombination von pharmakologischen Wirkungen:

1. Stimulierung und Wiederherstellung von Lernvermögen und Gedächtnis;
2. Wiederherstellung der Erregung im peripheren Nervensystem bei dessen Blockade durch verschiedene Faktoren (Entzündungen, Traumata, Antibiotikawirkung, Lokalanästhetika, Toxine, KCl);
3. Stimulierung der Nerven-Muskel-Übertragung und Anticurarewirkung;
4. Stimulierung der glatten Muskulatur und Verstärkung ihrer Reaktion auf Agonisten außer KCl;
5. bei der Wirkung auf das ZNS Kombination von stimulierenden mit sedativen Wirkungen;
6. antiarrhythmische Wirkung;
7. analgetische Eigenschaften.

Hinsichtlich des Ausprägungsgrades der Effekte des pharmakologischen Wirkungsspektrums im Vergleich mit den Kontrollpräparaten kann man die erfindungsgemäßen Verbindungen in folgende Gruppen unterteilen:

bicyclische Basen, die in der Hauptsache sämtliche für Amyridin kennzeichnenden Eigenschaften aufweisen, jedoch eine stärkere antiamnestische Wirkung zeigen;

Salze der tricyclischen Basen, die ein amyridinanaloges Wirkungsspektrum zeigen, jedoch in ausgeprägterem Maße die Skopolaminwirkung aufheben;

Salze der bicyclischen Basen, die bei amyridinanalogem Spektrum pharmakologischer Aktivität in weit höherem Maße die Lernfähigkeit und das Gedächtnis der Versuchstiere verbessern, und zwar in stärkerem Maße als die oben erwähnten Gruppen und ausgeprägtere antiarrhythmische und analgetische Wirkungen zeigen. Ferner sind sie stärkere AChE-Inhibitoren. Kennzeichnend für sie ist ferner der neue Effekt der direkten m-cholinomimetischen Wirkung auf Rezeptoren. Es ist darauf hinzuweisen, daß die Kombination der Blockade der $K^+$-Durchlässigkeit der Membrane mit der Inhibierung der Cholinesterase und M-cholinomimetischen Wirkung zu einer erheblichen Verstärkung der cholinergischen Übertragung führt, was besonders wichtig ist für die Behandlung von Gedächtnisschädigungen, insbesondere die Alzheimer Krankheit, bei der es zu einer starken Insuffizienz der cholinergischen Systeme des Gehirns kommt.

Nachfolgend werden Beispiele für die Herstellung der erfindungsgemäßen Verbindungen angeführt.

Beispiel 1

2-Methyl-3-ethyl-4-amino-6,7-dihydro-5H-1-pyrindinhydrat (21)

8 g (0,07 M) 2-Aminocyclopentencarbonitril werden mit 8,5 ml (0,08 M) Methylpropylketon und 50 g Polyphosphorsäure (PPS) versetzt und unter Erwärmen bei einer Temperatur von 70 bis 72° C 5 Stunden gerührt, wonach man das Reaktionsgemisch auf 40 bis 45° C abkühlt, in 5-fachem Volumen Wasser löst, mit Chlorofom extrahiert, den Chlorofomextrakt verwirft, die wäßrige Phase mit Ammoniak bis zu einem pH von 6 alkalisch stellt, den ausgefallenen Niederschlag des Nebenprodukts abfiltriert und verwirft. Das Filtrat wird mit einer wäßrigen $NH_3$-Lösung auf einen pH von 9 bis 10 eingestellt, wonach man den ausgefallenen Niederschlag des Endprodukts abfiltriert, die alkalische wäßrige Schicht mit Chloroform extrahiert, das Chloroform entfernt und den Niederschlag nach Abtrennung des Chloroforms mit dem im alkalischen Medium ausgefallenen Niederschlag vereinigt, diesen mit Wasser wäscht und trocknet. Die Ausbeute beträgt 1,4 g (10 %). Danach wird aus einem Gemisch von Alkohol und Wasser (1:1) umkristallisiert. Das erhaltene Produkt weist einen Fp von 140 bis 142° C auf.

Die Ergebnisse der Elementaranalyse sowie der IR-Spektroskopie sind in Tabelle 1 angegeben.

Beispiel 2

2-Ethyl-3-methyl-4-amino-6,7-dihydro-5H-1-pyrindinhydrat (27)

8 g (0,07 M) 2-Aminocyclopentencarbonitril werden mit 7 g (8,5 ml, 0,08 M) Diethylketon und 80 g PPS versetzt. Man rührt 5 Stunden bei einer Temperatur von 80 bis 85° C. Danach kühlt man das Reaktionsgemisch auf 60 bis 65° C ab, löst in 6-fachem Volumen Wasser, extrahiert mit Chloroform, trennt den Chloroformextrakt ab und verwirft ihn. Danach neutralisiert man die wäßrige Schicht mit einer Sodalösung bis zu einem pH von 6,5 bis 7, filtriert den ausgefallenen Niederschlag des Nebenprodukts ab, wäscht mit Wasser und verwirft ihn. Das Filtrat wird mit einer wäßrigen Ammoniaklösung bis zu einem pH von 9 bis 10 alkalisch gestellt. Nach zwei Stunden wird der ausgefallenen Niederschlag des Endprodukts filtriert, schnell mit Wasser gewaschen und getrocknet. Man erhält 5,75 g (40 %). Danach wird aus einem Gemisch von Propylalkohol und Wasser (1:2) umkristallisiert. Das erhaltene Produkt weist einen Fp von 147 bis 150° C auf. Die Ergebnisse der Elementaranalyse sowie der IR-Spektroskopie sind in Tabelle 1 angegeben.

Nachstehend folgt Tabelle 7, in welcher die Parameter sowie die Ausbeute des Endprodukts, das heißt der Verbindung (27) angegeben sind, die durch die Reaktion von 2-Aminocyclopentencarbonitril mit Diethylketon in Polyphosphorsäure in An-oder Abwesenheit eines Lösungsmittels erhalten wurde. Das Lösungsmittel wird dabei zur Stabilisierung der Temperaturverhältnisse eingesetzt.

Tabelle 7

| Beispiel Nr. | Molverhältnis von 2-Aminocyclopentencarbonitril zu Keton | Massenverhältnis von 2-Aminocyclopentencarbonitril zu PPS | Lösungsmittel | Reaktionstemperatur, °C | Reaktionsdauer, Stunden | Temperatur der Abkühlung, °C | Wasserüberschuß für die Lösung des Reaktionsgemisches, Teile | Organisches Lösungsmittel für die Extraktion | Alkali für die Neutralisierung der wäßrigen Schicht | Ausbeute an nicht umkristallisiertem Endprodukt | Lösungsmittel für die Umkristallisierung des Endproduktes (Verhältnis) |
|---|---|---|---|---|---|---|---|---|---|---|---|
| 1 | 2 | 3 | 4 | 5 | 6 | 7 | 8 | 9 | 10 | 11 | 12 |
| 3 | 1:1,3 | 1:7 | - | 60-65 | 5 | 30 | 6 | Chloroform | Sodalösung | 30 | Wasser: Isopropanol (1:1) |
| 4 | 1:1,2 | 1:12 | - | 80-85 | 3 | 40 | 6 | Ether | wäßriger Ammoniak | 42 | Wasser: Methanol (3:1) |
| 5 | 1:1,1 | 1:8 | - | 135-140 | 2 | 70 | 5 | Chloroform | 10%ige wäßrige KOH | 36 | Wasser: Methanol (4:1) |
| 6 | 1:1,3 | 1:10 | Toluol | 105-110 | 3 | 20 | 6 nach dem Lösen in Wasser wird die Toluol- | Chloroform | 15%ige Sodalösung | 35 | Wasser: Ethanol (5:1) |

Tabelle 7 (Fortsetzung)

| 1 | 2 | 3 | 4 | 5 | 6 | 7 | 8 | 9 | 10 | 11 | 12 |
|---|---|---|---|---|---|---|---|---|---|---|---|
| | | | | | | | schicht abgetrennt und verworfen | | | | |
| 7 | 1:1,2 | 1:9 | - | 50-55 | 6 | 50 | 4 | Chloroform | wäßriger Ammoniak | 25 | Alkohol:Ethylacetat:Wasser (1:5:0,1) |
| 8 | 1:1,3 | 1:8 | Benzol | 75-80 | 5 | 40 | 5 nach dem Lösen in Wasser wird die Benzolschicht abgetrennt und verworfen | Chloroform | wäßriger Ammoniak | 31 | Aceton:Alkohol:Wasser (2:1:1) |

Beispiel 9

2,3-Dimethyl-4-methylamino-6,7-dihydro-5H-1-pyrindinhydrat (19)

In einen Autoklaven werden 9 g (0,05 M) 2,3-Dimethyl-4-chlor-6,7-dihydro-5H-1-pyrindin gegeben, erhalten durch Diazotierung von 2,3-Dimethyl-4-amino-6,7-dihydro-5H-1-pyrindin mit Natriumnitrit in einer Lösung von 36 %-iger Salzsäure, wonach man 125 ml einer 25 %-igen wäßrigen Methylaminlösung und 25 ml Ethanol zusetzt und bei 210 °C 18 Stunden lang erwärmt. Das abgekühlte Reaktionsgemisch wird mit einer wäßrigen Ammoniaklösung bis zu einem pH von 9 bis 10 versetzt und mit Chloroform extrahiert. Aus dem Chloroformextrakt erhält man nach Entfernen des Chloroforms einen Rest, den man mit Ether (2x30 ml) wäscht, wodurch man einen kristallinen Niederschlag des Endprodukts erhält. Die Ausbeute beträgt 5,77 g (60 %). Nach Umkristallisieren aus einem Gemisch von Aceton, Alkohol und Wasser (1:0,5:1) beträgt der Fp 160 bis 162 °C. Die Ergebnisse der Elementaranalyse und der IR-Spektroskopie sind in Tabelle 1 angegeben.

Die Base 2,3-Dimethyl-4-ethylamino-6,7-dihydro-5H-1-pyrindin (20) erhält man analog zu obigem Verfahren aus 2,3-Dimethyl-4-Chlor-6,7-dihydro-5H-1-pyrindin und Ethylamin. Die übrigen Basen erhält man aus 2-Aminocyclopentencarbonitril und den entsprechenden Ketonen in PPS nach dem in Beispiel 2 beschriebenen Verfahren.

Beispiel 10

8-Amino-1,2,3,5,6,7-hexahydro-1-cyclopenta[b]pyrindin-hydrochlorid (36)

10,8 g (0,1 M) 2-Aminocyclopentencarbonitril werden mit 10,9 g (11,6 ml, 0,13 M) und 85 g PPS versetzt. Danach wird die Temperatur des Reaktionsgemisches vorsichtig auf 70 bis 75 °C angehoben, wonach man bei dieser Temperatur 5 Stunden lang rührt, dann auf 45 bis 50 °C abkühlt, das 6-fache Volumen Wasser zusetzt, bis zur Erzielung eines homogenen Gemisches rührt, mit Chloroform extrahiert (3x30 ml) und den Chloroformextrakt verwirft. Die wäßrige Schicht wird mit einer Sodalösung auf einen pH 6-7 neutralisiert, danach wird der ausgefallenen Niederschlag des Nebenprodukts filtriert, auf dem Filter mit Wasser gewaschen und verworfen. Das Filtrat wird mit wäßrigem Ammoniak auf einen pH von 9 bis 10 alkalisch gestellt. Nach 2 Stunden wird der ausgefallene Niederschlag der Base des Endprodukts filtriert, mit Wasser gewaschen und getrocknet. Die Ausbeute an Base, das heißt an 8-Amino-1,2,3,5,6,7-hexahydro-1-cyclopenta[b]pyrindin-monohydrat beträgt 2,9 g (15,2 %). Die erhaltene Base wird aus einem Gemisch von Isopropanol und Wasser (1:2) umkristallisiert. Fp: 198-200 °C.

Die erhaltene Base wird in einem minimalen Volumen Alkohol gelöst und mit einer alkoholischen Lösung von HCl auf einen pH von 3-3,5 eingestellt, wonach das Endprodukt mit Diethylether ausgefällt wird. Die Ausbeute beträgt 3,3 g (96 %). Nach der Umkristallisierung (Alkohol/Ethylacetat) weist das Produkt einen Schmelzpunkt von über 250 °C (unter Zers.) auf. Die Ergebnisse der Elementaranalyse sowie der IR-Spektroskopie sind in Tabelle 1 angegeben.

Beispiel 11

8-Amino-1,2,3,5,6,7-hexahydro-1-cyclopenta[b]pyrindinperchlorat (37)

Die Base des Endprodukts wird, wie in Beispiel 10 beschrieben, erhalten.

Die erhaltene Base wird dann in einem minimalen Volumen Alkohol gelöst und mit einer wäßrigen 57 %-igen Lösung von $HClO_4$ bis zu einem pH von 3,5 bis 4,0 versetzt. Danach wird das Endprodukt mit Aceton ausgefällt. Die Ausbeute beträgt 95 %. Zur Reinigung wird das Perchlorat mit Diethylether aus Alkohol umgefällt. Fp: 227 bis 230 °C.

Die Ergebnisse der Elementaranalyse sowie der IR-Spektroskopie sind in Tabelle 1 angegeben.

Analog zu Beispiel 10 erhält man die Verbindungen 38 und 39.

## Beispiel 12

4-Amino-2,3-dimethyl-6,7-dihydro-5H-1-pyrindinhydrochlorid (1)

9,5 g (0,053 M) der Base 4-Amino-2,3-dimethyl-6,7-dihydro-5H-1-pyrindinmonohydrat werden in 15 ml Alkohol (Methanol, Ethanol, Isopropanol) gelöst. Dieser Lösung wird eine Lösung von HCl in abs. Ethylalkohol bis zu einem pH von 3,5 bis 4 bei einer Temperatur von 15 bis 20 °C zugetropft. Das erhalttene Salz wird mit 100 ml Ehter ausgefällt, filtriert und mit Ether gewaschen. Der Niederschlag wird aus Alkohol mit Ether umgefällt. Die Ausbeute beträgt 10,2 g (98 %).

## Beispiel 13

4-Amino-2,3-dimethyl-6,7-dihydro-5H-1-pyrindinsalicylat (8)

8,5 (0,047 M) der Base 4-Amino-2,3-dimethyl-6,7-dihydro-5H-1-pyrindinmonohydrat werden in 12 ml Methanol gelöst. Die erhaltene Lösung wird mit 6,5 g (0,047 M) Salicylsäure in 10 ml Alkohol versetzt. Nach der Entfernung des Alkohols wird der Rückstand mit Aceton trituriert. Die Ausbeute beträgt 13,3 g (95 %).

## Beispiel 14

4-Amino-2,3-dimethyl-6,7-dihydro-5H-1-pyrindinfumarat (12)

7,7 g (0,043 M) der Base 4-Amino-2,3-dimethyl-6,7-dihydro-5H-1-pyrindinmonohydrat werden in 12 ml Methanol gelöst. Die erhaltene Lösung wird mit einer Lösung von 5 g (0,043 M) Fumarsäure in 12 ml Alkohol versetzt. Nach Entfernen des Alkohols wird der Rückstand mit Aceton trituriert und mit Aceton aus Methanol umgefällt. Die Ausbeute beträgt 11,42 g (96 %).

Die übrigen Salze werden analog zu dem oben beschriebenen Verfahren aus der entsprechenden Base und einer Säure erhalten. Die Ausbeuten sowie die physikalischen und chemischen Eigenschaften der Salze sind in Tabelle 1 angegeben.

Außerdem werden noch Arzneimittelpräparate vorgeschlagen, die als Wirkstoff 4-Amino-2,3-disubst.-6,7-dihydro-5H-1-pyrindinderivate enthalten und nach den üblichen Verfahren unter Verwendung der in der Medizin gebräuchlichen Zusätze hergestellt werden. Solche Arzneimittel verwendet man vorzugsweise in Form von Injektionslösungen und Tabletten.

## Ansprüche

1. 4-Amino-2,3- disubst.-6,7-dihydro-5H-1-pyrindinderivate der allgemeinen Formel

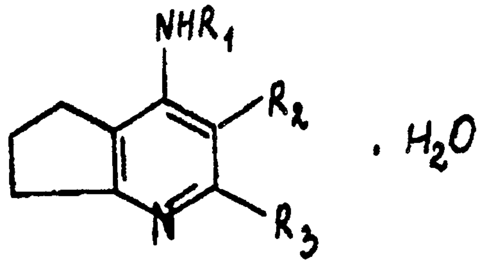

in welcher $R_1$ Wasserstoff oder Niederalkyl,
$R_2$ Niederalkyl oder Phenyl und
$R_3$ Niederalkyl,
mit der Ausnahme, daß $R_2$ = $R_3$ = $CH_3$ und
$R_1$ Wasserstoff ist,
bedeuten.

2. 4-Amino-2,3- disubst.-6,7-dihydro-5H-1-pyrindinderivate der allgemeinen Formel

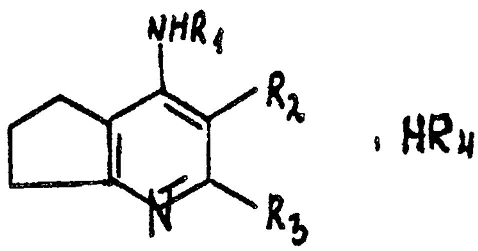

in welcher $R_1$ Wasserstoff bedeutet,
$(R_2 + R_3) = (-CH_2-)_n$, wobei n 3 oder 5 ist und
$HR_4$ HCl oder $HClO_4$ bedeutet.

3. 4-Amino-2,3- disubst.-6,7-dihydro-5H-1-pyrindinderivate der allgemeinen Formel

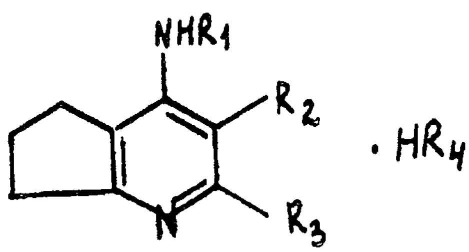

in welcher $R_1$ Wasserstoff,
$R_2$ Niederalkyl oder Phenyl,
$R_3$ Niederalkyl und
$HR_4$ eine Säure, ausgewählt aus der Gruppe der anorganischen und organischen Säuren der aliphatischen oder aromatischen Reihe,
bedeutet.

4. 4-Amino-2,3- disubst.-6,7-dihydro-5H-1-pyrindinderivate nach Anspruch 1, worin $R_1$ ein Rest ist, ausgewählt aus der Gruppe $CH_3$ oder $C_2H_5$.

5. 4-Amino-2,3- disubst.-6,7-dihydro-5H-1-pyrindinderivate nach den Ansprüchen 1 und 3, worin $R_2$ ausgewählt ist aus der Gruppe $CH_3$, $C_2H_5$, $C_3H_7$, $C_4H_9$ oder $C_6H_5$.

6. 4-Amino-2,3- disubst.-6,7-dihydro-5H-1-pyrindinderivate nach Anspruch 5, worin der Rest $R_2$ $CH_3$ oder $C_2H_5$ ist.

7. 4-Amino-2,3- disubst.-6,7-dihydro-5H-1-pyrindinderivate nach Anspruch 6, worin $R_2$ $CH_3$ ist.

8. 4-Amino-2,3- disubst.-6,7-dihydro-5H-1-pyrindinderivate nach den Ansprüchen 1 und 3, worin $R_3$ ausgewählt ist aus der Gruppe $CH_3$, $C_2H_5$ oder $C_4H_9$.

9. 4-Amino-2,3- disubst.-6,7-dihydro-5H-1-pyrindinderivate nach Anspruch 8, worin $R_3$ $CH_3$ ist.

10. 4-Amino-2,3- disubst.-6,7-dihydro-5H-1-pyrindinderivate nach Anspruch 3, worin $R_4$ ein Rest einer anorganischen Säure, welcher die Elemente V - VII der Gruppe 1 - 3 des Periodensystems sowie Br- oder J- umfaßt, ist.

11. 4-Amino-2,3- disubst.-6,7-dihydro-5H-1-pyrindinderivate nach Anspruch 3, worin die anorganische Säure ausgewählt ist aus der Gruppe HCl, $HClO_4$, HBr, $H_2SO_4$ oder $H_3PO_4$.

12. 4-Amino-2,3- disubst.-6,7-dihydro-5H-1-pyrindinderivate nach Anspruch 11, worin die anorganische Säure ausgewählt ist aus der Gruppe HCl, $HClO_4$, HBr oder $H_2SO_4$.

13. 4-Amino-2,3- disubst.-6,7-dihydro-5H-1-pyrindinderivate nach Anspruch 3, worin die organische Säure der aliphatischen oder aromatischen Reihe eine Monocarbonsäure, ausgewählt aus der Gruppe Essig-, Capron-, Capryl-, Palmitin-, Stearin-, Benzoe-, o-Toluyl- oder Nikotinsäure, ist.

14. 4-Amino-2,3- disubst.-6,7-dihydro-5H-1-pyrindinderivate nach Anspruch 13, worin die organische Säure der aliphatischen oder aromatischen Reihe eine Monocarbonsäure, ausgewählt aus der Gruppe Essig-, Capron-, Capryl- oder Nikotinsäure, ist.

15. 4-Amino-2,3- disubst.-6,7-dihydro-5H-1-pyrindinderivate nach Anspruch 14, worin die organische aliphatische oder aromatische Säure eine Monocarbonsäure ist, ausgewählt aus der Gruppe der Essig- oder Nikotinsäure.

16. 4-Amino-2,3- disubst.-6,7-dihydro-5H-1-pyrindinderivate nach Anspruch 3, worin die organische Säure der aliphatischen oder aromatischen Reihe eine ungesättigte Säure ist, ausgewählt aus der Gruppe Malein-, Fumar- oder Zimtsäure.

17. 4-Amino-2,3- disubst.-6,7-dihydro-5H-1-pyrindinderivate nach Anspruch 16, worin die ungesättigte organische Säure Zimtsäure ist.

18. 4-Amino-2,3- disubst.-6,7-dihydro-5H-1-pyrindinderivate nach Anspruch 3, worin die organische Säure der aliphatischen Reihe eine Dicarbonsäure ist, ausgewählt aus der Gruppe Oxal-, Malon-, Bernstein- oder Adipinsäure.

19. 4-Amino-2,3- disubst.-6,7-dihydro-5H-1-pyrindinderivate nach Anspruch 18, worin die organische Dicarbonsäure der aliphatischen Reihe Oxal- oder Adipinsäure ist.

20. 4-Amino-2,3- disubst.-6,7-dihydro-5H-1-pyrindinderivate nach Anspruch 19, worin die organische Dicarbonsäure der aliphatischen Reihe Adipinsäure ist.

21. 4-Amino-2,3- disubst.-6,7-dihydro-5H-1-pyrindinderivate nach Anspruch 3, worin die organische Säure der aliphatischen oder aromatischen Reihe eine Mono-, Di- oder Tri-Oxysäure ist, ausgewählt aus der Gruppe Salicyl-, Wein- oder Zitronensäure.

22. 4-Amino-2,3- disubst.-6,7-dihydro-5H-1-pyrindinderivate nach Anspruch 21, worin die organische Oxysäure der aliphatischen oder aromatischen Reihe Salicyl- oder Weinsäure ist.

23. 4-Amino-2,3- disubst.-6,7-dihydro-5H-1-pyrindinderivate nach Anspruch 22, worin die organische Oxysäure Weinsäure ist.

24. 4-Amino-2,3- disubst.-6,7-dihydro-5H-1-pyrindinderivate nach Anspruch 3, worin die organische Aminosäure der aliphatischen oder aromatischen Reihe Glutaminsäure ist.

25. Verfahren zur Herstellung von 4-Amino-2,3- disubst.-6,7-dihydro-5H-1-pyrindinderivaten nach Anspruch 1 durch Umsetzung von 2-Aminocyclopentencarbonitril mit einem Keton bei einem Molarverhältnis von 1:1,1-1,3 in Gegenwart von Polyphosphorsäure, dadurch **gekennzeichnet**, daß man die Reaktion in einem 7- bis 12-fachen Überschuß von Polyphosphorsäure in bezug auf 2-Amino-cyclopentencarbonitril bei 50 bis 140 °C während 2 bis 5 Stunden durchführt, wonach man das auf 20 bis 80 °C abgekühlte Reaktionsgemisch mit Wasser (1:4-6) verdünnt, drei Mal mit Ether oder Chloroform extrahiert, die organische Schicht abtrennt, die zurückgebliebene wäßrige Schicht mit Alkali bis zu einem pH von 6-7 neutralisiert, den ausgefallenen Niederschlag abfiltriert, das Filtrat mit einer wäßrigen Ammoniaklösung bis zu einem pH von 9-10 behandelt, den erhaltenen Niederschlag der Base abfiltriert und aus einem organischen Lösungsmittel unter Zugabe von Wasser umkristallisiert, wobei bei $R_1$ = Niederalkyl die erhaltene Base zu den Pyridin-4-Chlorderivaten diazotiert wird, die man dann aminoalkyliert.

26. Verfahren zur Herstellung von 4-Amino-2,3- disubst.-6,7-dihydro-5H-1-pyrindinderivate nach Anspruch 2 durch Umsetzung von 2-Aminocyclopentencarbonitril mit einem Keton bei einem Molarverhältnis von 1:1,1-1,3 in Gegenwart von Polyphosphorsäure bis zur Bildung einer Base, die mit der entsprechenden Säure umgesetzt wird, dadurch **gekennzeichnet**, daß die Reaktion in einem 7- bis 12-fachen Überschuß von Polyphosphorsäure in bezug auf 2-Aminocyclopentencarbonitril bei 50 bis 140 °C während 2 bis 5 Stunden durchgeführt wird, wonach man das auf 20 bis 80 °C abgekühlte Reaktionsgemisch mit Wasser

(1:4-6) verdünnt, drei Mal mit Ether oder Chloroform extrahiert, die organische Schicht abtrennt, die zurückgebliebene wäßrige Schicht mit Alkali bis zu einem pH von 6-7 neutralisiert, den ausgefallenen Niederschlag abfiltriert, das Filtrat mit einer wäßrigen Ammoniaklösung bis zu einem pH von 9-10 behandelt, den erhaltenen Niederschlag der Base abfiltriert und aus einem organischen Lösungsmittel unter Zugabe von Wasser umkristallisiert und die auf diese Weise erhaltene Base mit HCl oder $HClO_4$ in an sich bekannter Weise umsetzt.

27. Verfahren zur Herstellung von 4-Amino-2,3- disubst.-6,7-dihydro-5H-1-pyrindinderivaten nach Anspruch 3 durch Umsetzung von 2-Aminocyclopentencarbonitril mit einem Keton bei einem Molarverhältnis von 1:1,1-1,3 in Gegenwart von Polyphosphorsäure bis zur Bildung einer Base, die mit der entsprechenden Säure umgesetzt wird, dadurch **gekennzeichnet**, daß die Reaktion in einem 7- bis 12-fachen Überschuß von Polyphosphorsäure in bezug auf 2-Aminocyclopentencarbonitril bei 50 bis 140°C während 2 bis 5 Stunden durchgeführt wird, wonach man das auf 20 bis 80°C abgekühlte Reaktionsgemisch mit Wasser (1:4-6) verdünnt, drei Mal mit Ether oder Chloroform extrahiert, die organische Schicht abtrennt, die zurückgebliebene wäßrige Schicht mit Alkali bis zu einem pH von 6-7 neutralisiert, den ausgefallenen Niederschlag abfiltriert, das Filtrat mit einer wäßrigen Ammoniaklösung bis zu einem pH von 9-10 behandelt, den erhaltenen Niederschlag der Base abfiltriert und aus einem organischen Lösungsmittel unter Zugabe von Wasser umkristallisiert und die auf diese Weise erhaltene Base mit Säuren, ausgewählt aus der Gruppe der anorganischen oder organischen Säuren der aliphatischen oder aromatischen Reihe in an sich bekannter Weise umsetzt.

28. Arzneimittelpräparat, dadurch **gekennzeichnet**, daß es als Wirkstoff 4-Amino-2,3- disubst.-6,7-dihydro-5H-1-pyrindinderivate nach Anspruch 1 enthält.

29. Arzneimittelpräparat, dadurch **gekennzeichnet**, daß es als Wirkstoff 4-Amino-2,3- disubst.-6,7-dihydro-5H-1-pyrindinderivate nach Anspruch 2 enthält.

30. Arzneimittelpräparat, dadurch **gekennzeichnet**, daß es als Wirkstoff 4-Amino-2,3- disubst.-6,7-dihydro-5H-1-pyrindinderivate nach einem der Ansprüche 3 bis 24 enthält.

31. Arzneimittelpräparat nach Anspruch 30, dadurch **gekennzeichnet**, daß es als Wirkstoff das 2,3-Dimethyl-4-amino-6,7-dihydro-5H-1-pyrindinsulfat enthält.

Europäisches Patentamt

# EUROPÄISCHER RECHERCHENBERICHT

Nummer der Anmeldung: EP 89101353.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 97, Nr. 25, 20. Dezember 1982, Columbus, Ohio, USA<br>N.D.GRIGOREVA et al. "2,3-Substituted-4-aminopyrindines"<br>Seite 839, Spalte 1, Zusammenfassung-Nr. 216 034q<br>& SU-A-615 653; Otkrytia, Izobret., Prom. Obraztsy, Tovarnye Znaki 1982(17), 275<br>-- | 1,2 | C 07 D 221/04<br>C 07 D 221/16<br>A 61 K 31/435 |
| A | US - A - 2 333 493 (RIGBY)<br>* Anspruch 5; Beispiele I,IV; Seite 6, Spalte 1, Zeilen 39-46 *<br>---- | 1,25, 28 | |
| | | | **RECHERCHIERTE SACHGEBIETE (Int. Cl.4)** |
| | | | C 07 D 221/00 |

Der vorliegende Recherchenbericht wurde für alle Patentansprüche erstellt.

| Recherchenort | Abschlußdatum der Recherche | Prüfer |
|---|---|---|
| WIEN | 28-04-1989 | HOCHHAUSER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument